(19) Europäisches Patentamt
European Patent Office
Office européen des brevets

(11) Veröffentlichungsnummer: **0 554 729 B1**

# (12) EUROPÄISCHE PATENTSCHRIFT

(45) Veröffentlichungstag der Patentschrift: **06.12.95**

(51) Int. Cl.[6]: **C07C 333/04**, C07C 327/40, A01N 47/10, A01N 37/18

(21) Anmeldenummer: **93100967.4**

(22) Anmeldetag: **22.01.93**

## (54) Substituierte Aminosäureamide.

(30) Priorität: **04.02.92 DE 4203084**

(43) Veröffentlichungstag der Anmeldung:
**11.08.93 Patentblatt 93/32**

(45) Bekanntmachung des Hinweises auf die Patenterteilung:
**06.12.95 Patentblatt 95/49**

(84) Benannte Vertragsstaaten:
**BE CH DE ES FR GB IT LI NL PT**

(56) Entgegenhaltungen:
**EP-A- 0 398 072**
**CH-A- 655 305**

**CHEMICAL AND PHARMACEUTICAL BULLETIN, Band 19, Nr. 5, Mai 1971, Tokyo K.ISHIKAWA et al. "Studies on Optically Active Amino Acids. XIX. Solvent Effects on Optical Rotatory Dispersion and Circular Dichroism of N-Dithio-carbethoxy-L-alpha-amino Acids", Seiten 912-929**

(73) Patentinhaber: **BAYER AG**
**D-51368 Leverkusen (DE)**

(72) Erfinder: **Seitz, Thomas, Dr.**
**Mozartstrasse 32**
**W-4019 Monheim (DE)**
Erfinder: **Dehne, Heinz-Wilelm, Dr.**
**Krischer Strasse 81**
**W-4019 Monheim (DE)**

**EP 0 554 729 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Aminosäureamide, ein Verfahren zu ihrer Herstellung und ihre Verwendung als Schädlingsbekämpfungsmittel.

Es ist bekannt, daß bestimmte substituierte Aminosäureamide wie beispielsweise die Verbindung $N^{\alpha}$-(t-Butyloxycarbonyl)-L-valin-[N-(1-phenylethyl)-amid] oder die Verbindung $N^{\alpha}$-(t-Butyloxycarbonyl)-L-valin-{N-[1-(2-chlorphenyl)ethyl]-amid} oder die Verbindung $N^{\alpha}$-(t-Butyloxycarbonyl)-L-valin-{N-methyl-N-[1-(4-chlorphenyl)-ethyl]-amid} fungizide Eigenschaften besitzen (vgl. z.B. EP 398 072).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Aminosäureamide der allgemeinen Formel (I),

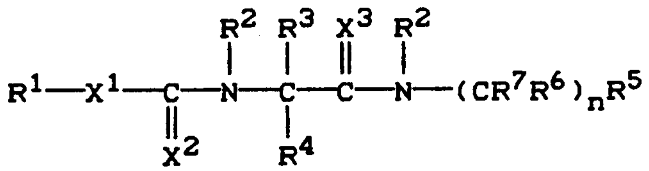
(I)

in welcher

| | |
|---|---|
| $R^1$ | für Alkyl, Alkenyl, Alkinyl, Halogenalkyl, Halogenalkenyl, Halogenalkinyl, Cycloalkyl, Cycloalkenyl oder für jeweils gegebenenfalls substituiertes Arylalkyl, Aryl, Heteroarylalkyl oder Heteroaryl steht, |
| $R^2$ | für Wasserstoff, Alkyl oder Cycloalkyl steht, |
| $R^3$ | für Wasserstoff, Alkyl oder Cycloalkyl steht und |
| $R^4$ | für Alkyl oder Cycloalkyl steht oder |
| $R^3$ und $R^4$ | gemeinsam mit dem Kohlenstoffatom, an welches sie gebunden sind, für einen Cycloalkylring stehen, |
| $R^5$ | für jeweils gegebenenfalls substituiertes Cycloalkyl, Cycloalkenyl, Aryl, Heterocyclyl, Cycloalkylalkyl, Cycloalkenylalkyl, Arylalkyl oder Heterocyclylalkyl steht, |
| $R^7$, $R^6$ | unabhängig voneinander für Wasserstoff oder für jeweils gegebenenfalls substituiertes Alkyl, Alkenyl, Alkinyl, Cycloalkyl, Cycloalkenyl, Aryl, Heterocyclyl, Cycloalkylalkyl, Cycloalkenylalkyl, Arylalkyl oder Heterocyclylalkyl stehen oder zusammen einen Cycloalkylring bilden, |
| n | für eine Zahl O, 1, 2 oder 3 steht, |
| $X^1$ | für Sauerstoff oder Schwefel steht, |
| $X^2$ | für Sauerstoff oder Schwefel steht und |
| $X^3$ | für Sauerstoff oder Schwefel steht, |

wobei jedoch mindestens einer der Substituenten $X^1$, $X^2$ und/oder $X^3$ für Schwefel steht, gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Aminosäureamide der allgemeinen Formel (I),

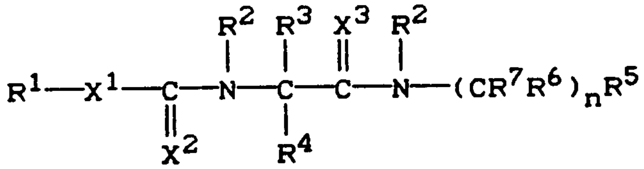
(I)

in welcher

n, $R^1$, $R^2$, $R^3$, $R^4$, $R^5$, $R^6$, $R^7$, $X^1$, $X^2$ und $X^3$ die oben angegebene Bedeutung haben,

erhält, wenn man substituierte Aminosäuren der Formel (II),

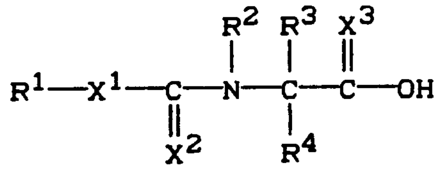

(II)

in welcher

$R^1$, $R^2$, $R^3$, $R^4$, $X^1$, $X^2$ und $X^3$ die oben angegebene Bedeutung haben,

mit Aminen der Formel (III),

$R^2HN—(CR^7R^6)_nR^5$ (III)

in welcher

n, $R^2$, $R^5$, $R^6$ und $R^7$ die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen substituierten Aminosäureamide der allgemeinen Formel (I) eine gute Wirksamkeit gegen Schädlinge besitzen.

Überraschenderweise zeigen die erfindungsgemäßen substituierten Aminosäureamide der allgemeinen Formel (I) eine erheblich bessere fungizide Wirksamkeit als die aus dem Stand der Technik bekannten substituierten Aminosäureamide, wie beispielsweise die Verbindung $N^{\alpha}$-(t-Butyloxycarbonyl)-L-valin-[N-(1-phenylethyl)-amid] oder die Verbindung $N^{\alpha}$-(t-Butyloxycarbonyl)-L-valin-{N-[1-(2-chlorphenyl)ethyl]-amid} oder die Verbindung $N^{\alpha}$-(t-Butyloxycarbonyl)-L-valin-{N-methyl-N-[1-(4-chlorphenyl)-ethyl]-amid}, welche chemisch und wirkungsmäßig naheliegende Verbindungen sind.

Die erfindungsgemäßen substituierten Aminosäureamide sind durch die Formel (I) allgemein definiert. Bevorzugt sind Verbindungen der Formel (I), bei welchen

$R^1$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 6 Kohlenstoffatomen und 1 bis 13 gleichen oder verschiedenen Halogenatomen, Halogenalkenyl mit 2 bis 6 Kohlenstoffatomen und 1 bis 11 gleichen oder verschiedenen Halogenatomen, Halogenalkinyl mit 2 bis 6 Kohlenstoffatomen und 1 bis 11 gleichen oder verschiedenen Halogenatomen, Cycloalkyl mit 3 bis 8 Kohlenstoffatomen, Cycloalkenyl mit 3 bis 8 Kohlenstoffatomen, oder für jeweils gegebenenfalls im Aryl- bzw. Heteroarylteil einfach oder mehrfach, gleich oder verschieden substituiertes Arylalkyl, Aryl, Heteroarylalkyl oder Heteroaryl mit jeweils gegebenenfalls 6 bis 10 Kohlenstoffatomen im Arylteil bzw. 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 6 Kohlenstoffatomen im geradkettigen oder versweigten Alkylteil steht, wobei als Aryl- bzw. Heteroarylsubstituenten jeweils infrage kommen:

Halogen, Cyano, Nitro, Hydroxy, Formyl, Hydroxycarbonyl, Aminocarbonyl, Aminosulfonyl, jeweils geradkettiges oder verzweigtes Alkyl, Alkoxy, Alkoxyalkyl, Alkylthio, Alkylsulfinyl, Alkylsulfonyl, Alkoxysulfonyl, Alkylsulfonyloxy oder Dialkylamino mit jeweils 1 bis 4 Kohlenstoffatomen in den einzelnen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkylcarbonyl, Alkylcarbonyloxy, Alkoxycarbonyl, N-Alkylaminocarbonyl, N,N-Dialkylaminocarbonyl, Alkylcarbonylamino, Alkylcarbonylaminoalkyl oder Alkoximinoalkyl mit jeweils 1 bis 4 Kohlenstoffatomen in den einzelnen Alkylteilen sowie jeweils gegebenenfalls im Phenylteil einfach oder mehrfach, gleich oder verschieden durch Halogen und/oder geradkettiges oder verzweigtes Alkyl mit 1 bis 4 Kohlenstoffatomen substituiertes Phenyl, Phenoxy, Phenylcarbonyl, Phenoxycarbonyl oder Phenylcarbonyloxy,

$R^2$ für Wasserstoff, für geradkettiges oder verzweigtes Alkyl mit 1 bis 8 Kohlenstoffatomen oder für Cycloalkyl mit 3 bis 8 Kohlenstoffatomen steht,

$R^3$ für Wasserstoff, für geradkettiges oder verzweigtes Alkyl mit 1 bis 8 Kohlenstoffatomen oder für Cycloalkyl mit 3 bis 8 Kohlenstoffatomen steht und

$R^4$ für geradkettiges oder verzweigtes Alkyl mit 1 bis 8 Kohlenstoffatomen oder für Cycloalkyl mit 3 bis 8 Kohlenstoffatomen steht oder

$R^3$ und $R^4$ gemeinsam mit dem Kohlenstoffatom, an welches sie gebunden sind, für einen 3- bis 8-gliedrigen Cycloalkylring stehen,

$R^5$ für jeweils gegebenenfalls einfach oder mehrfach, gleich oder verschieden substituiertes Cycloalkyl, Cycloalkylalkyl, Cycloalkenyl oder Cycloalkenylalkyl mit jeweils 3 bis 8 Kohlenstoffatomen steht, wobei als Substituenten jeweils infrage kommen: Halogen, Hydroxy, jeweils geradkettiges oder verzweigtes Alkyl, Alkandiyl, Alkoxy, Alkoxyalkyl, Alkylthio oder Dialkylamino mit jeweils 1 bis 6 Kohlenstoffatomen in den einzelnen Alkylteilen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen,

außerdem für jeweils gegebenenfalls einfach oder mehrfach, gleich oder verschieden substituiertes Aryl oder Arylalkyl mit 6 bis 10 Kohlenstoffatomen oder Heterocyclyl oder Heterocyclylalkyl mit 2 bis 9 Kohlenstoffatomen und 1 bis 4 gleichen oder verschiedenen Heteroatomen - insbesondere Stickstoff, Sauerstoff und/oder Schwefel - steht, wobei als Substituenten jeweils die bei $R^1$ genannten infrage kommen,

$R^7$, $R^6$ unabhängig voneinander für Wasserstoff, für jeweils geradkettiges oder verzweigtes Alkyl mit 1 bis 8 Kohlenstoffatomen, Cyanalkyl mit 2 bis 9 Kohlenstoffatomen, Alkenyl mit 2 bis 8 Kohlenstoffatomen, Alkinyl mit 2 bis 8 Kohlenstoffatomen oder für jeweils gegebenenfalls im Cycloalkyl- bzw. Cycloalkenylteil einfach oder mehrfach, gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil steht, wobei als Cycloalkyl- bzw. Cycloalkenylsubstituenten jeweils infrage kommen:

Halogen, Hydroxy, jeweils geradkettiges oder verzweigtes Alkyl, Alkandiyl, Alkoxy, Alkoxyalkyl, Alkylthio oder Dialkylamino mit jeweils 1 bis 6 Kohlenstoffatomen in den einzelnen Alkylteilen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen,

außerdem für jeweils gegebenenfalls im Aryl- bzw. Heterocyclylteil einfach oder mehrfach, gleich oder verschieden substituiertes Arylalkyl, Aryl, Heterocyclylalkyl oder Heterocyclyl mit jeweils gegebenenfalls 6 bis 10 Kohlenstoffatomen im Arylteil bzw. 2 bis 9 Kohlenstoffatomen und 1 bis 4 gleichen oder verschiedenen Heteroatomen - insbesondere Stickstoff, Sauerstoff und/oder Schwefel - im Heterocyclylteil und gegebenenfalls 1 bis 6 Kohlenstoffatomen im geradkettigen oder versweigten Alkylteil steht, wobei als Aryl- bzw. Heterocyclylsubstituenten jeweils die bei $R^1$ genannten infrage kommen, oder zusammen einen Cycloalkylring mit 3 bis 7 Kohlenstoffatomen bilden,

n für eine Zahl 0, 1, 2 oder 3 steht,

$X^1$ für Sauerstoff oder Schwefel steht,

$X^2$ für Sauerstoff oder Schwefel steht und

$X^3$ für Sauerstoff oder Schwefel steht,

wobei jedoch mindestens einer der Substituenten $X^1$, $X^2$ und/oder $X^3$ für Schwefel steht.

Besonders bevorzugt sind Verbindungen der Formel (I), bei welchen

$R^1$ für jeweils geradkettiges oder verzweigtes Alkyl mit 1 bis 6 Kohlenstoffatomen, Alkenyl mit 2 bis 6 Kohlenstoffatomen, Alkinyl mit 2 bis 6 Kohlenstoffatomen, Halogenalkyl mit 1 bis 4 Kohlenstoffatomen und 1 bis 9 gleichen oder verschiedenen Halogenatomen, Halogenalkenyl mit 2 bis 4 Kohlenstoffatomen und 1 bis 7 gleichen oder verschiedenen Halogenatomen, Halogenalkinyl mit 2 bis 4 Kohlenstoffatomen und 1 bis 7 gleichen oder verschiedenen Halogenatomen, Cycloalkyl mit 3 bis 7 Kohlenstoffatomen, Cycloalkenyl mit 3 bis 7 Kohlenstoffatomen, oder für jeweils gegebenenfalls im Aryl- bzw. Heteroarylteil einfach bis fünffach, gleich oder verschieden substituiertes Arylalkyl, Aryl, Heteroarylalkyl oder Heteroaryl mit jeweils gegebenenfalls 6 bis 10 Kohlenstoffatomen im Arylteil bzw. 2 bis 9 Kohlenstoffatomen und 1 bis 3 gleichen oder verschiedenen Heteroatomen - insbesondere Stickstoff, Sauerstoff und/oder Schwefel - im Heteroarylteil und gegebenenfalls 1 bis 4 Kohlenstoffatomen im geradkettigen oder verzweigten Alkylteil steht, wobei als Aryl- bzw.

Heteroarylsubstituenten jeweils infrage kommen:
Halogen, Cyano, Nitro, Hydroxy, Formyl, Hydroxycarbonyl, Aminocarbonyl, Aminosulfonyl, jeweils geradkettiges oder verzweigtes Alkyl, Alkoxy, Alkoxyalkyl, Alkylthio, Alkylsulfinyl, Alkylsulfonyl, Alkoxysulfonyl, Alkylsulfonyloxy oder Dialkylamino mit jeweils 1 bis 4 Kohlenstoffatomen in den einzelnen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkylcarbonyl, Alkylcarbonyloxy, Alkoxycarbonyl, N-Alkylaminocarbonyl, N,N-Dialkylaminocarbonyl, Alkylcarbonylamino, Alkylcarbonylaminoalkyl oder Alkoximinoalkyl mit jeweils 1 bis 4 Kohlenstoffatomen in den einzelnen Alkylteilen sowie jeweils gegebenenfalls im Phenylteil einfach oder mehrfach, gleich oder verschieden durch Halogen und/oder geradkettiges oder verzweigtes Alkyl mit 1 bis 4 Kohlenstoffatomen substituiertes Phenyl, Phenoxy, Phenylcarbonyl, Phenoxycarbonyl oder Phenylcarbonyloxy,

$R^2$ für Wasserstoff, für geradkettiges oder verzweigtes Alkyl mit 1 bis 6 Kohlenstoffatomen oder für Cycloalkyl mit 3 bis 7 Kohlenstoffatomen steht,

$R^3$ für Wasserstoff, für geradkettiges oder verzweigtes Alkyl mit 1 bis 6 Kohlenstoffatomen oder für Cycloalkyl mit 3 bis 7 Kohlenstoffatomen steht und

$R^4$ für geradkettiges oder verzweigtes Alkyl mit 1 bis 6 Kohlenstoffatomen oder für Cycloalkyl mit 3 bis 6 Kohlenstoffatomen steht oder

$R^3$ und $R^4$ gemeinsam mit dem Kohlenstoffatom, an welches sie gebunden sind, für einen 3- bis 7-gliedrigen Cycloalkylring stehen,

$R^5$ für jeweils gegebenenfalls einfach bis fünffach, gleich oder verschieden substituiertes Cycloalkyl, Cycloalkylalkyl, Cycloalkenylalkyl oder Cycloalkenyl mit jeweils 3 bis 7 Kohlenstoffatomen steht, wobei als Cycloalkyl- bzw. Cycloalkenylsubstituenten jeweils infrage kommen:
Halogen, Hydroxy, jeweils geradkettiges oder verzweigtes Alkyl, Alkandiyl, Alkoxy, Alkoxyalkyl, Alkylthio oder Dialkylamino mit jeweils 1 bis 4 Kohlenstoffatomen in den einzelnen Alkylteilen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen,
außerdem für jeweils gegebenenfalls einfach bis fünffach, gleich oder verschieden substituiertes Aryl oder Arylalkyl mit 6 bis 10 Kohlenstoffatomen oder Heterocyclyl oder Heterocyclylalkyl mit 2 bis 9 Kohlenstoffatomen und 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils die bei $R^1$ genannten infrage kommen,

$R^7$, $R^6$ unabhängig voneinander für Wasserstoff, für jeweils geradkettiges oder verzweigtes Alkyl mit 1 bis 6 Kohlenstoffatomen, Cyanalkyl mit 2 bis 5 Kohlenstoffatomen, Alkenyl mit 2 bis 6 Kohlenstoffatomen, Alkinyl mit 2 bis 6 Kohlenstoffatomen oder für jeweils gegebenenfalls im Cycloalkyl- bzw. Cycloalkenylteil einfach bis fünffach, gleich oder verschieden substituiertes Cycloalkyl, Cycloalkylalkyl, Cycloalkenyl oder Cycloalkenylalkyl mit jeweils 3 bis 7 Kohlenstoffatomen im Cycloalkyl- bzw. Cycloalkenylteil und gegebenenfalls 1 bis 4 Kohlenstoffatomen im geradkettigen oder verzweigten Alkylteil steht, wobei als Cycloalkyl- bzw. Cycloalkenylsubstituenten infrage kommen:
Halogen, Hydroxy, jeweils geradkettiges oder verzweigtes Alkyl, Alkandiyl, Alkoxy, Alkoxyalkyl, Alkylthio oder Dialkylamino mit jeweils 1 bis 4 Kohlenstoffatomen in den einzelnen Alkylteilen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen,
außerdem für jeweils gegebenenfalls im Aryl- bzw. Heterocyclylteil einfach bis fünffach, gleich oder verschieden substituiertes Arylalkyl, Aryl, Heterocyclylalkyl oder Heterocyclyl mit jeweils gegebenenfalls 6 bis 10 Kohlenstoffatomen im Arylteil bzw. 2 bis 9 Kohlenstoffatomen und 1 bis 3 gleichen oder verschiedenen Heteroatomen - insbesondere Stickstoff, Sauerstoff und/oder Schwefel - im Heterocyclylteil und gegebenenfalls 1 bis 4 Kohlenstoffatomen im geradkettigen oder verzweigten Alkylteil steht, wobei als Aryl- bzw. Heterocyclylsubstituenten jeweils die bei $R^1$ genannten infrage kommen, oder zusammen einen Cycloalkylring mit 3 bis 6 Kohlenstoffatomen bilden,

n für eine Zahl 0, 1, 2 oder 3 steht,

$X^1$ für Sauerstoff oder Schwefel steht,
$X^2$ für Sauerstoff oder Schwefel steht und
$X^3$ für Sauerstoff oder Schwefel steht,

wobei jedoch mindestens einer der Substituenten $X^1$, $X^2$ und/oder $X^3$ für Schwefel steht.

Ganz besonders bevorzugt sind Verbindungen der Formel (I), bei welchen

$R^1$ für jeweils geradkettiges oder verzweigtes Alkyl mit 2 bis 5 Kohlenstoffatomen, Alkenyl mit 2 bis 4 Kohlenstoffatomen, Alkinyl mit 2 bis 4 Kohlenstoffatomen, Halogenalkyl mit 1 oder 2 Kohlenstoffatomen und 1 bis 5 gleichen oder verschiedenen Halogenatomen, Halogenalkenyl mit 2 bis 3 Kohlenstoffatomen und 1 bis 5 gleichen oder verschiedenen Halogenatomen, Halogenalkinyl mit 2 bis 3 Kohlenstoffatomen und 1 bis 5 gleichen oder verschiedenen Halogenatomen, Cycloalkyl mit 3 bis 6 Kohlenstoffatomen, Cycloalkenyl mit 3 bis 6 Kohlenstoffatomen, oder für jeweils gegebenenfalls im Phenylteil einfach bis dreifach, gleich oder verschieden substituiertes Phenyl oder Phenylalkyl mit gegebenenfalls 1 bis 4 Kohlenstoffatomen im geradkettigen oder verzweigten Alkylteil steht, wobei als Phenylsubstituenten jeweils infrage kommen:
Halogen, Cyano, Nitro, Hydroxy, Formyl, Hydroxycarbonyl, Aminocarbonyl, Aminosulfonyl, jeweils geradkettiges oder verzweigtes Alkyl, Alkoxy, Alkoxyalkyl, Alkylthio, Alkylsulfinyl, Alkylsulfonyl, Alkoxysulfonyl, Alkylsulfonyloxy oder Dialkylamino mit jeweils 1 bis 3 Kohlenstoffatomen in den einzelnen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 oder 2 Kohlenstoffatomen und 1 bis 5 gleichen oder verschiedenen Halogenatomen, jeweils geradkettiges oder verzweigtes Alkylcarbonyl, Alkylcarbonyloxy, Alkoxycarbonyl, N-Alkylaminocarbonyl, N,N-Dialkylaminocarbonyl, Alkylcarbonylamino, Alkylcarbonylaminoalkyl oder Alkoximinoalkyl mit jeweils 1 bis 3 Kohlenstoffatomen in den einzelnen Alkylteilen sowie jeweils gegebenenfalls im Phenylteil einfach oder mehrfach, gleich oder verschieden durch Halogen und/oder geradkettiges oder verzweigtes Alkyl mit 1 bis 3 Kohlenstoffatomen substituiertes Phenyl, Phenoxy, Phenylcarbonyl, Phenoxycarbonyl oder Phenylcarbonyloxy,

$R^2$ für Wasserstoff, für geradkettiges oder verzweigtes Alkyl mit 1 bis 4 Kohlenstoffatomen oder für Cycloalkyl mit 3 bis 6 Kohlenstoffatomen steht,

$R^3$ für Wasserstoff, für geradkettiges oder verzweigtes Alkyl mit 1 bis 4 Kohlenstoffatomen oder für Cycloalkyl mit 3 bis 6 Kohlenstoffatomen steht und

$R^4$ für geradkettiges oder verzweigtes Alkyl mit 1 bis 4 Kohlenstoffatomen oder für Cycloalkyl mit 3 bis 6 Kohlenstoffatomen steht oder

$R^3$ und $R^4$ gemeinsam mit dem Kohlenstoffatom, an welches sie gebunden sind, für einen 3- bis 6-gliedrigen Cycloalkylring stehen,

$R^5$ für jeweils gegebenenfalls einfach bis dreifach, gleich oder verschieden substituiertes Cycloalkyl, Cycloalkylalkyl, Cycloalkenylalkyl oder Cycloalkenyl mit jeweils 3 bis 6 Kohlenstoffatomen steht, wobei als Substituenten jeweils infrage kommen:
Halogen, Hydroxy, jeweils geradkettiges oder verzweigtes Alkyl, Alkandiyl, Alkoxy, Alkoxyalkyl, Alkylthio oder Dialkylamino mit jeweils 1 bis 3 Kohlenstoffatomen in den einzelnen Alkylteilen oder jeweils geradkettiges oder verzweigtes Halogenalkyl, Halogenalkoxy, Halogenalkylthio mit jeweils 1 oder 2 Kohlenstoffatomen und 1 bis 5 gleichen oder verschiedenen Halogenatomen,
außerdem für jeweils gegebenenfalls einfach bis dreifach, gleich oder verschieden substituiertes Phenyl oder Phenylalkyl oder Heterocyclyl oder Heterocyclylalkyl mit 2 bis 9 Kohlenstoffatomen und 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils die bei $R^1$ genannten infrage kommen,

$R^7$, $R^6$ unabhängig voneinander für Wasserstoff, für jeweils geradkettiges oder verzweigtes Alkyl mit 1 bis 4 Kohlenstoffatomen, Cyanalkyl mit 2 bis 3 Kohlenstoffatomen, Alkenyl mit 2 bis 4 Kohlenstoffatomen, Alkinyl mit 2 bis 4 Kohlenstoffatomen oder für jeweils gegebenenfalls im Cycloalkyl- bzw. Cycloalkenylteil einfach bis dreifach, gleich oder verschieden substituiertes Cycloalkyl, Cycloalkylalkyl, Cycloalkenyl oder Cycloalkenylalkyl mit jeweils 3 bis 6 Kohlenstoffatomen im Cycloalkyl- bzw. Cycloalkenylteil und gegebenenfalls 1 bis 3 Kohlenstoffatomen im geradkettigen oder verzweigten Alkylteil steht, wobei als Cycloalkyl- bzw. Cycloalkenylsubstituenten jeweils infrage kommen:
Halogen, Hydroxy, jeweils geradkettiges oder verzweigtes Alkyl, Alkandiyl, Alkoxy, Alkoxyalkyl, Alkylthio oder Dialkylamino mit jeweils 1 bis 3 Kohlenstoffatomen in den einzelnen Alkylteilen oder jeweils geradkettiges oder verzweigtes Halogenalkyl, Halogenalkoxy, Halogenalkylthio mit jeweils 1 oder 2 Kohlenstoffatomen und 1 bis 5 gleichen oder verschiedenen Halogenatomen,
außerdem für jeweils gegebenenfalls im Phenyl- bzw. Heterocyclylteil einfach bis dreifach, gleich oder verschieden substituiertes Phenylalkyl, Phenyl, Heterocyclylalkyl oder Heterocyclyl mit jeweils gegebenenfalls 2 bis 9 Kohlenstoffatomen und 1 bis 3 gleichen oder verschiedenen Heteroatomen - insbesondere Stickstoff, Sauerstoff und/oder Schwefel - im Heterocyclylteil und gegebenenfalls 1 bis 3 Kohlenstoffatomen im geradkettigen oder verzweigten Alkylteil steht, wobei als Aryl- bzw. Heterocyclylsubstituenten jeweils die bei $R^1$ genannten infrage kommen, oder zusammen einen Cycloalkylring mit 3 bis 5 Kohlenstoffatomen bilden,

n für eine Zahl 1 oder 2 steht,
$X^1$ für Sauerstoff oder Schwefel steht,
$X^2$ für Sauerstoff oder Schwefel steht und
$X^3$ für Sauerstoff oder Schwefel steht,

wobei jedoch mindestens einer der Substituenten $X^1$, $X^2$ und/oder $X^3$ für Schwefel steht.

Im einzelnen sei auf die bei den Herstellungsbeispielen genannten Verbindungen verwiesen.

Verwendet man beispielsweise $N^\alpha$-(i-Propylthio-carbonyl)-L-valin und $\alpha$-(4-Methylphenyl)-ethylamin als Ausgangsstoffe, so läßt sich der Reaktionsablauf des erfindungsgemäßen Verfahrens durch das folgende Formelschema darstellen:

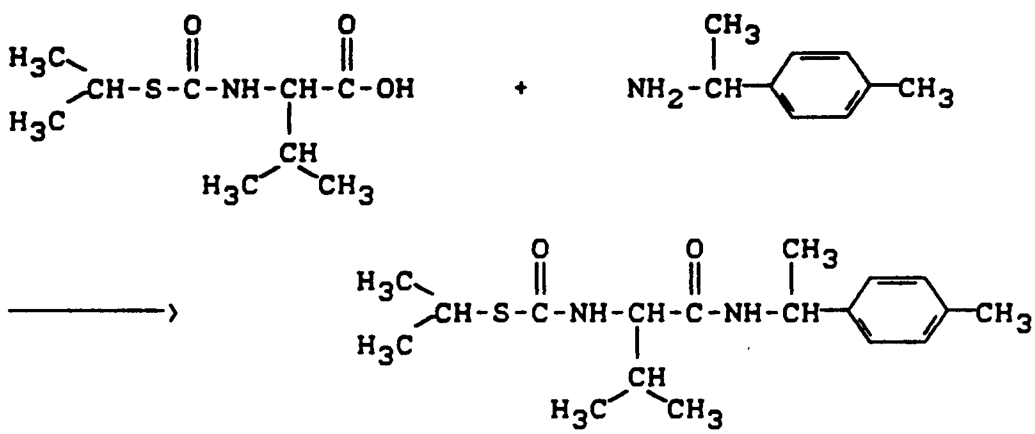

Die zur Durchführung des erfindungsgemäßen Verfahrens als Ausgangsstoffe benötigten substituierten Aminosäuren sind durch die Formel (II) allgemein definiert. In dieser Formel (II) stehen $R^1$, $R^2$, $R^3$, $R^4$, $X^1$, $X^2$ und $X^3$ vorzugsweise für diejenigen Reste, die bereits im Zusammenhang mit der Beschreibung der erfindungsgemäßen Stoffe der Formel (I) als bevorzugt für diese Substituenten genannt wurden. Die substituierten Aminosäuren der Formel (II) sind bekannt oder lassen sich in Analogie zu bekannten Verfahren herstellen (vgl. z.B. Houben-Weyl "Methoden der organischen Chemie" Band 15/1, 4. Auflage, Thieme Verlag Stuttgart 1977; JP 53148530; JP 52151146; J. Org. Chem. 43, 2930-2932 [1978]; J, Chem. Soc., Perkin Trans. 1, 1972, 1983-1985; Chem. Ber. 104, 3156-3167 [1971]; J. Org. Chem. 36, 49-59 [1971]; Helv. Chim. Acta 52, 282-291 [1969]; Tetrahedron 34, 2763-2766 [1978]; Chem. Pharm. Bull. 19, 912-929 [1971]; J, Chem, Soc. 1952, 2076-2079).

Die zur Durchführung des erfindungsgemäßen Verfahrens als Ausgangsstoffe benötigten Amine sind durch die Formel (III) allgemein definiert. In dieser Formel (III) stehen $R^5$ und $R^6$ vorzugsweise für diejenigen Reste, die bereits im Zusammenhang mit der Beschreibung der erfindungsgemäßen Stoffe der Formel (I) als bevorzugt für diese Substituenten genannt wurden.
Die Amine der Formel (III) sind allgemein bekannte Verbindungen der organischen Chemie oder erhältlich in Analogie zu allgemein bekannten Verfahren.

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether

wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Sulfoxide wie Dimethylsulfoxid.

Das erfindungsgemäße Verfahren wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner, wie Phosphortribromid, Phosphorpentachlorid, Phosphoroxychlorid oder Thionylchlorid, Anhydridbildner, wie Chlorameisensäureethylester, Chlorameisensäureisobutylester oder Methansulfonylchlorid, Carbodiimide, wie N,N'-Dicyclohexylcarbodiimid (DCC) oder andere übliche Kondensationsmittel, wie N,N'-Carbonyldiimidazol, 2-Ethoxy-N-ethoxycarbonyl-1,2-dihydrochinolin (EEDQ) oder Triphenylphosphin/Tetrachlorkohlenstoff.

Das erfindungsgemäße Verfahren wird vorzugsweise in Gegenwart eines geeigneten Reaktionshilfsmittels durchgeführt, Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden, Im allgemeinen arbeitet man bei Temperaturen zwischen -60 °C und 220 °C, vorzugsweise bei Temperaturen zwischen -20 °C und 100 °C.

Zur Durchführung des erfindungsgemäßen Verfahrens setzt man pro Mol an substituierter Aminosäure der Formel (II) im allgemeinen 1,0 bis 10,0 Mol, vorzugsweise 1,0 bis 2,0 Mol an Amin der Formel (III) und gegebenenfalls - 1,0 bis 3,0 Mol, vorzugsweise 1,0 bis 2,0 Mol an Kondensationsmittel bzw. Reaktionshilfsmittel ein. Die Reaktionsdurchführung, Aufarbeitung und Isolierung der Reaktionsprodukte erfolgt nach allgemein üblichen Methoden (vgl. hierzu beispielsweise Houben Weyl "Methoden der organischen Chemie", Band 15/2, 4. Auflage, Thieme Verlag Stuttgart 1977 oder die Herstellungsbeispiele). Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phythophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;

Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen den Erreger der Tomatenbraunfäule (Phytophthora infestans) eingesetzt werden.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester. Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den

Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele:

Beispiel 1:

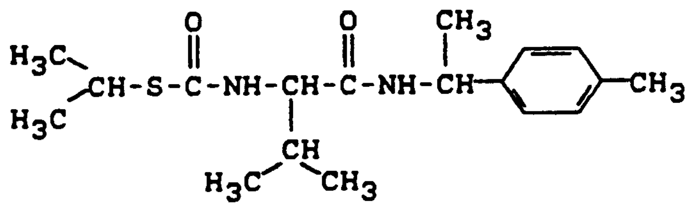

Zu 3,28 g (0,015 mol) $N^{\alpha}$-(i-Propylthio-carbonyl)-L-valin in 50 ml Dichlormethan gibt man bei -20 °C 1,5 g (0,015 Mol) N-Methylpiperidin und anschließend tropfenweise unter Rühren bei -20 °C 2,0 g (0,015 Mol) Chlorameisensäure-isobutylester, rührt nach beendeter Zugabe weitere 10 Minuten bei -20 °C, kühlt dann auf -60 °C ab und gibt 2,0 g (0,015 Mol) $\alpha$-(4-Methylphenyl)-ethylamin zu, wobei die Temperatur -15 °C nicht überschreiten soll. Anschließend rührt man weitere 2 Stunden bei -15 °C und 15 Stunden bei Raumtemperatur, filtriert dann ausgefallenen Feststoff ab, wäscht mit Dichlormethan nach, engt das Filtrat im Vakuum ein, nimmt den Rückstand in Wasser auf, extrahiert mehrfach mit Essigester, wäscht die vereinigten Essigester-Phasen mit wäßriger Natriumhydrogencarbonatlösung und Wasser, trocknet über Natriumsulfat und entfernt das Lösungsmittel im Vakuum.

Man erhält 2,8 g (58% der Theorie) an $N^{\alpha}$-(i-Propylthio-carbonyl)-L-valin-[N-(4-Methylphenyl)-ethylamid] vom Schmelzpunkt 168 °C.

Herstellung der Ausgangsverbindung:

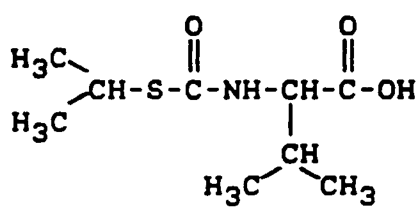

Zu 37,5 g (0,32 Mol) L-Valin und 88,0 g (0,64 Mol) Kaliumcarbonat in 400 ml Wasser gibt man tropfenweise unter Rühren und Eiskühlung 45 g (0,32 Mol) Chlorameisensäureisopropylthioester, rührt nach beendeter Zugabe eine weitere Stunde bei 0 °C, säuert dann mit konzentrierter Salzsäure an, extrahiert dreimal mit jeweils 200 ml Toluol, trocknet die vereinigten organischen Phasen über Natriumsulfat und entfernt das Lösungsmittel im Vakuum. Das verbleibende zähe Öl kristallisiert nach einiger Zeit.

Man erhält 37,2 g (53% der Theorie) an $N^{\alpha}$-(i-Propylthio-carbonyl)-L-valin vom Schmelzpunkt 65 °C.

In entsprechender Weise und gemäß den allgemeinen Angaben zur Herstellung erhält man die folgenden substituierten Aminosäureamide der allgemeinen Formel (I):

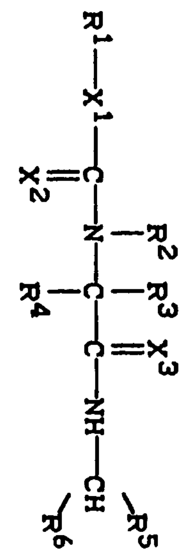

(I)

| Bsp.-Nr. | $R^1$ | $R^2$ | $R^3$ | $R^4$ | $R^5$ | $R^6$ | $X^1$ | $X^2$ | $X^3$ | Fp./°C*) |
|---|---|---|---|---|---|---|---|---|---|---|
| 2 | $i-C_3H_7$ | H | H | $i-C_3H_7$ | $CH_3$ | 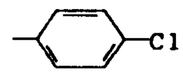 | S | O | O | 189 (rac.) |
| 3 | $i-C_3H_7$ | H | H | $i-C_3H_7$ | $CH_3$ | 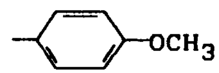 | S | O | O | 170 (rac.) |
| 4 | $i-C_3H_7$ | H | H | $i-C_3H_7$ | $CH_3$ | 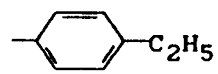 | S | O | O | 148 (rac.) |
| 5 | $i-C_3H_7$ | H | H | $i-C_3H_7$ | $CH_3$ | $C_6H_5$ | S | O | O | 179 |
| 6 | $i-C_3H_7$ | H | H | $i-C_3H_7$ | $CH_3$ | 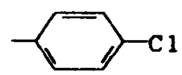 | S | O | O | 191 (R+) |
| 7 | $i-C_3H_7$ | H | H | $i-C_3H_7$ | $CH_3$ | $C_6H_5$ | S | O | O | 199 (R+) |
| 8 | $i-C_3H_7$ | H | H | $i-C_3H_7$ | $CH_3$ | $C_6H_5$ | S | O | O | 176 (rac.) |
| 9 | $i-C_3H_7$ | H | H | $i-C_3H_7$ | $CH_3$ | 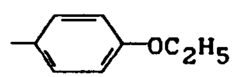 | S | O | O | 196 (rac.) |

| Bsp.-Nr. | $R^1$ | $R^2$ | $R^3$ | $R^4$ | $R^5$ | $R^6$ | $X^1$ | $X^2$ | $X^3$ | Fp./°C*) |
|---|---|---|---|---|---|---|---|---|---|---|
| 10 | $i-C_3H_7$ | H | H | $i-C_3H_7$ | $CH_3$ | 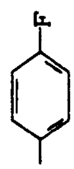 | S | O | O | 204 (rac.) |
| 11 | $t-C_4H_9$ | H | H | $i-C_3H_7$ | $CH_3$ | 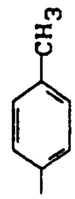 | S | O | O | 179 (rac.) |

*) Die Angaben zur Stereochemie beziehen sich jeweils auf die Aminkomponente im Amidteil. Als Ausgangsverbindungen der Formel (II) wurden ausschließlich L-Aminosäurederivate eingesetzt.

Anwendungsbeispiele:

In dem folgenden Anwendungsbeispiel wurden die nachstehend aufgeführten Verbindungen als Vergleichssubstanzen eingesetzt:

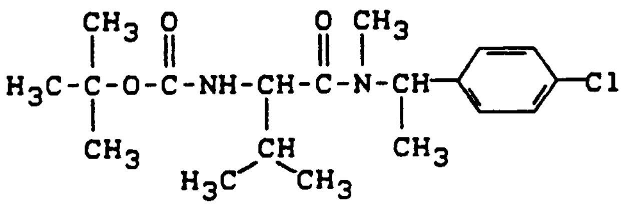

(A)

$N^{\alpha}$-(t-Butyloxycarbonyl)-L-valin-{N-methyl-N-[1-(4-chlorphenyl)-ethyl]-amid}

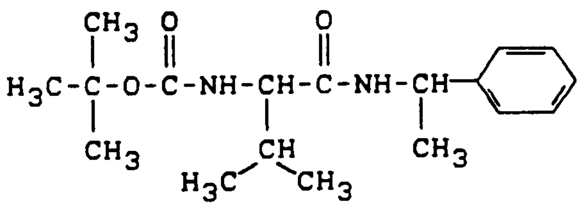

(B)

$N^{\alpha}$-(t-Butyloxycarbonyl)-L-valin-[N-(1-phenylethyl)-amid]

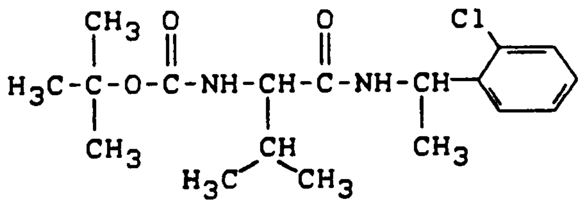

(C)

$N^{\alpha}$-(t-Butyloxycarbonyl)-L-valin-{N-[1-(2-chlorphenyl)-ethyl]-amid}
(alle bekannt aus EP 398 072)

Beispiel A:

Phytophthora-Test (Tomate)/protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden dann in einer Inkubationskabine bei 20 °C und einer relativen Luftfeuchtigkeit von ca. 100% aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 2, 3, 4, 5, 7, 10 und 11, welche bei einer Wirkstoffkonzentration von 100 ppm eine 100 %igen Wirkungsgradzeigen.

## Patentansprüche

**1.** Aminosäureamide der Formel (I),

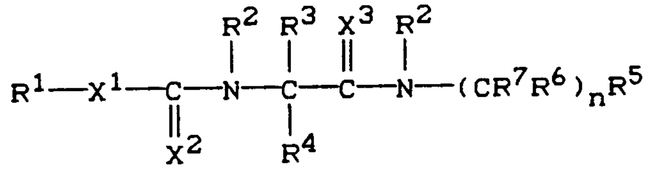 (I)

in welcher

| | |
|---|---|
| $R^1$ | für Alkyl, Alkenyl, Alkinyl, Halogenalkyl, Halogenalkenyl, Halogenalkinyl, Cycloalkyl, Cycloalkenyl oder für jeweils gegebenenfalls substituiertes Arylalkyl, Aryl, Heteroarylalkyl oder Heteroaryl steht, |
| $R^2$ | für Wasserstoff, Alkyl oder Cycloalkyl steht, |
| $R^3$ | für Wasserstoff, Alkyl oder Cycloalkyl steht und |
| $R^4$ | für Alkyl oder Cycloalkyl steht oder |
| $R^3$ und $R^4$ | gemeinsam mit dem Kohlenstoffatom, an welches sie gebunden sind, für einen Cycloalkylring stehen, |
| $R^5$ | für jeweils gegebenenfalls substituiertes Cycloalkyl, Cycloalkenyl, Aryl, Heterocyclyl, Cycloalkylalkyl, Cycloalkenylalkyl, Arylalkyl oder Heterocyclylalkyl steht, |
| $R^7$, $R^6$ | unabhängig voneinander für Wasserstoff oder für jeweils gegebenenfalls substituiertes Alkyl, Alkenyl, Alkinyl, Cycloalkyl, Cycloalkenyl, Aryl, Heterocyclyl, Cycloalkylalkyl, Cycloalkenylalkyl, Arylalkyl oder Heterocyclylalkyl stehen, oder zusammen einen Cycloalkylring bilden, |
| n | für eine Zahl 0, 1, 2 oder 3 steht, |
| $X^1$ | für Sauerstoff oder Schwefel steht, |
| $X^2$ | für Sauerstoff oder Schwefel steht und |
| $X^3$ | für Sauerstoff oder Schwefel steht, |

wobei jedoch mindestens einer der Substituenten $X^1$, $X^2$ und/oder $X^3$ für Schwefel steht,

**2.** Aminosäureamide der Formel (I) gemäß Anspruch 1, bei welchen

| | |
|---|---|
| $R^1$ | für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 6 Kohlenstoffatomen und 1 bis 13 gleichen oder verschiedenen Halogenatomen, Halogenalkenyl mit 2 bis 6 Kohlenstoffatomen und 1 bis 11 gleichen oder verschiedenen Halogenatomen, Halogenalkinyl mit 2 bis 6 Kohlenstoffatomen und 1 bis 11 gleichen oder verschiedenen Halogenatomen, Cycloalkyl mit 3 bis 8 Kohlenstoffatomen, Cycloalkenyl mit 3 bis 8 Kohlenstoffatomen, oder für jeweils gegebenenfalls im Aryl- bzw. Heteroarylteil einfach oder mehrfach, gleich oder verschieden substituiertes Arylalkyl, Aryl, Heteroarylalkyl oder Heteroaryl mit jeweils gegebenenfalls 6 bis 10 Kohlenstoffatomen im Arylteil bzw. 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 6 Kohlenstoffatomen im geradkettigen oder verzweigten Alkylteil steht, wobei als Aryl- bzw. Heteroarylsubstituenten jeweils infrage kommen:<br>Halogen, Cyano, Nitro, Hydroxy, Formyl, Hydroxycarbonyl, Aminocarbonyl, Aminosulfonyl, jeweils geradkettiges oder verzweigtes Alkyl, Alkoxy, Alkoxyalkyl, Alkylthio, Alkylsulfinyl, Alkylsulfonyl, Alkoxysulfonyl, Alkylsulfonyloxy oder Dialkylamino mit jeweils 1 bis 4 Kohlenstoffatomen in den einzelnen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkylcarbonyl, Alkylcarbonyloxy, Alkoxycarbonyl, N-Alkylaminocarbonyl, N,N-Dialkylaminocarbonyl, Alkylcarbonylamino, Alkylcarbonylaminoalkyl oder Alkoximinoalkyl mit jeweils 1 bis 4 Kohlenstoffatomen in den einzelnen Alkylteilen sowie jeweils gegebenenfalls |

im Phenylteil einfach oder mehrfach, gleich oder verschieden durch Halogen und/oder geradkettiges oder verzweigtes Alkyl mit 1 bis 4 Kohlenstoffatomen substituiertes Phenyl, Phenoxy, Phenylcarbonyl, Phenoxycarbonyl oder Phenylcarbonyloxy,

$R^2$ für Wasserstoff, für geradkettiges oder verzweigtes Alkyl mit 1 bis 8 Kohlenstoffatomen oder für Cycloalkyl mit 3 bis 8 Kohlenstoffatomen steht,

$R^3$ für Wasserstoff, für geradkettiges oder verzweigtes Alkyl mit 1 bis 8 Kohlenstoffatomen oder für Cycloalkyl mit 3 bis 8 Kohlenstoffatomen steht und

$R^4$ für geradkettiges oder verzweigtes Alkyl mit 1 bis 8 Kohlenstoffatomen oder für Cycloalkyl mit 3 bis 8 Kohlenstoffatomen steht oder

$R^3$ und $R^4$ gemeinsam mit dem Kohlenstoffatom, an welches sie gebunden sind, für einen 3- bis 8-gliedrigen Cycloalkylring stehen,

$R^5$ für jeweils gegebenenfalls einfach oder mehrfach, gleich oder verschieden substituiertes Cycloalkyl oder Cycloalkylalkyl oder Cycloalkenyl oder Cycloalkenylalkyl mit jeweils 3 bis 8 Kohlenstoffatomen steht, wobei als Substituenten jeweils infrage kommen:

Halogen, Hydroxy, jeweils geradkettiges oder verzweigtes Alkyl, Alkandiyl, Alkoxy, Alkoxyalkyl, Alkylthio oder Dialkylamino mit jeweils 1 bis 6 Kohlenstoffatomen in den einzelnen Alkylteilen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen,

außerdem für jeweils gegebenenfalls einfach oder mehrfach, gleich oder verschieden substituiertes Aryl oder Arylalkyl mit 6 bis 10 Kohlenstoffatomen oder Heterocyclyl oder Heterocyclylalkyl mit 2 bis 9 Kohlenstoffatomen und 1 bis 4 gleichen oder verschiedenen Heteroatomen - insbesondere Stickstoff, Sauerstoff und/oder Schwefel - steht, wobei als Aryl- bzw. Heterocyclylsubstituenten jeweils die bei $R^1$ genannten infrage kommen,

$R^7$, $R^6$ unabhängig voneinander für Wasserstoff, für jeweils geradkettiges oder verzweigtes Alkyl mit 1 bis 8 Kohlenstoffatomen, Cyanalkyl mit 2 bis 9 Kohlenstoffatomen, Alkenyl mit 2 bis 8 Kohlenstoffatomen, Alkinyl mit 2 bis 8 Kohlenstoffatomen oder für jeweils gegebenenfalls im Cycloalkyl- bzw. Cycloalkenylteil einfach oder mehrfach, gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil steht, wobei als Cycloalkyl- bzw. Cycloalkenylsubstituenten jeweils infrage kommen:

Halogen, Hydroxy, jeweils geradkettiges oder verzweigtes Alkyl, Alkandiyl, Alkoxy, Alkoxyalkyl, Alkylthio oder Dialkylamino mit jeweils 1 bis 6 Kohlenstoffatomen in den einzelnen Alkylteilen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen,

außerdem für jeweils gegebenenfalls im Aryl- bzw. Heterocyclylteil einfach oder mehrfach, gleich oder verschieden substituiertes Arylalkyl, Aryl, Heterocyclylalkyl oder Heterocyclyl mit jeweils gegebenenfalls 6 bis 10 Kohlenstoffatomen im Arylteil bzw. 2 bis 9 Kohlenstoffatomen und 1 bis 4 gleichen oder verschiedenen Heteroatomen - insbesondere Stickstoff, Sauerstoff und/oder Schwefel - im Heterocyclylteil und gegebenenfalls 1 bis 6 Kohlenstoffatomen im geradkettigen oder verzweigten Alkylteil steht, wobei als Aryl- bzw. Heterocyclylsubstituenten jeweils die bei $R^1$ genannten infrage kommen, oder zusammen einen Cycloalkylring mit 3 bis 7 Kohlenstoffatomen bilden,

n für eine Zahl 0, 1, 2 oder 3 steht,

$X^1$ für Sauerstoff oder Schwefel steht,

$X^2$ für Sauerstoff oder Schwefel steht und

$X^3$ für Sauerstoff oder Schwefel steht,

wobei jedoch mindestens einer der Substituenten $X^1$, $X^2$ und/oder $X^3$ für Schwefel steht.

**3.** Verbindungen der Formel (I) gemäß Anspruch 1, bei welchen

$R^1$ für jeweils geradkettiges oder verzweigtes Alkyl mit 1 bis 6 Kohlenstoffatomen, Alkenyl mit 2 bis 6 Kohlenstoffatomen, Alkinyl mit 2 bis 6 Kohlenstoffatomen, Halogenalkyl mit 1 bis 4 Kohlenstoffatomen und 1 bis 9 gleichen oder verschiedenen Halogenatomen, Halogenalkenyl mit 2 bis 4 Kohlenstoffatomen und 1 bis 7 gleichen oder verschiedenen Halogenatomen, Halogenalkinyl mit 2 bis 4 Kohlenstoffatomen und 1 bis 7 gleichen oder verschiedenen Halogenatomen, Cycloalkyl mit 3 bis 7 Kohlenstoffatomen, Cycloalkenyl mit 3 bis 7 Kohlenstoffatomen, oder für jeweils gegebenenfalls im Aryl- bzw. Heteroarylteil einfach bis fünffach, gleich oder verschieden substituiertes Arylalkyl, Aryl, Heteroarylalkyl oder Heteroaryl mit jeweils gegebenenfalls 6 bis 10 Kohlenstoffatomen im Arylteil bzw. 2 bis 9 Kohlenstoffatomen und 1 bis 3 gleichen oder verschiedenen Heteroatomen - insbesondere Stickstoff, Sauerstoff und/oder Schwefel - im Heteroarylteil und gegebenenfalls 1 bis 4 Kohlenstoffatomen im geradkettigen oder verzweigten Alkylteil steht, wobei als Aryl- bzw. Heteroarylsubstituenten jeweils infrage kommen:

Halogen, Cyano, Nitro, Hydroxy, Formyl, Hydroxycarbonyl, Aminocarbonyl, Aminosulfonyl, jeweils geradkettiges oder verzweigtes Alkyl, Alkoxy, Alkoxyalkyl, Alkylthio, Alkylsulfinyl, Alkylsulfonyl, Alkoxysulfonyl, Alkylsulfonyloxy oder Dialkylamino mit jeweils 1 bis 4 Kohlenstoffatomen in den einzelnen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkylcarbonyl, Alkylcarbonyloxy, Alkoxycarbonyl, N-Alkylaminocarbonyl, N,N-Dialkylaminocarbonyl, Alkylcarbonylamino, Alkylcarbonylaminoalkyl oder Alkoximinoalkyl mit jeweils 1 bis 4 Kohlenstoffatomen in den einzelnen Alkylteilen sowie jeweils gegebenenfalls im Phenylteil einfach oder mehrfach, gleich oder verschieden durch Halogen und/oder geradkettiges oder verzweigtes Alkyl mit 1 bis 4 Kohlenstoffatomen substituiertes Phenyl, Phenoxy, Phenylcarbonyl, Phenoxycarbonyl oder Phenylcarbonyloxy,

$R^2$ für Wasserstoff, für geradkettiges oder verzweigtes Alkyl mit 1 bis 6 Kohlenstoffatomen oder für Cycloalkyl mit 3 bis 7 Kohlenstoffatomen steht,

$R^3$ für Wasserstoff, für geradkettiges oder verzweigtes Alkyl mit 1 bis 6 Kohlenstoffatomen oder für Cycloalkyl mit 3 bis 7 Kohlenstoffatomen steht und

$R^4$ für geradkettiges oder verzweigtes Alkyl mit 1 bis 6 Kohlenstoffatomen oder für Cycloalkyl mit 3 bis 6 Kohlenstoffatomen steht oder

$R^3$ und $R^4$ gemeinsam mit dem Kohlenstoffatom, an welches sie gebunden sind, für einen 3-bis 7-gliedrigen Cycloalkylring stehen,

$R^5$ für jeweils gegebenenfalls einfach bis fünffach, gleich oder verschieden substituiertes Cycloalkyl, Cycloalkylalkyl, Cycloalkenylalkyl oder Cycloalkenyl mit jeweils 3 bis 7 Kohlenstoffatomen steht, wobei als Substituenten jeweils infrage kommen:

Halogen, Hydroxy, jeweils geradkettiges oder verzweigtes Alkyl, Alkandiyl, Alkoxy, Alkoxyalkyl, Alkylthio oder Dialkylamino mit jeweils 1 bis 4 Kohlenstoffatomen in den einzelnen Alkylteilen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen,

außerdem für jeweils gegebenenfalls einfach bis fünffach, gleich oder verschieden substituiertes Aryl oder Arylalkyl mit 6 bis 10 Kohlenstoffatomen oder Heterocyclyl oder Heterocyclylalkyl mit 2 bis 9 Kohlenstoffatomen und 1 bis 3 gleichen oder verschiedenen Heteroatomen - insbesondere Stickstoff, Sauerstoff und/oder Schwefel -steht, wobei als Substituenten jeweils die bei $R^1$ genannten infrage kommen,

$R^7$, $R^6$ unabhängig voneinander für Wasserstoff, für jeweils geradkettiges oder verzweigtes Alkyl mit 1 bis 6 Kohlenstoffatomen, Cyanalkyl mit 2 bis 5 Kohlenstoffatomen, Alkenyl mit 2 bis 6 Kohlenstoffatomen, Alkinyl mit 2 bis 6 Kohlenstoffatomen oder für jeweils gegebenenfalls im Cycloalkyl- bzw. Cycloalkenylteil einfach bis fünffach, gleich oder verschieden substituiertes Cycloalkyl, Cycloalkylalkyl, Cycloalkenyl oder Cycloalkenylalkyl mit jeweils 3 bis 7 Kohlenstoffatomen im Cycloalkyl- bzw. Cycloalkenylteil und gegebenenfalls 1 bis 4 Kohlenstoffatomen im geradkettigen oder verzweigten Alkylteil steht, wobei als Cycloalkyl- bzw. Cycloalkenylsubstituenten infrage kommen:

Halogen, Hydroxy, jeweils geradkettiges oder verzweigtes Alkyl, Alkandiyl, Alkoxy, Alkoxyalkyl, Alkylthio oder Dialkylamino mit jeweils 1 bis 4 Kohlenstoffatomen in den einzelnen Alkylteilen oder jeweils geradkettiges oder verzweigtes Halogenalkyl, Halogenalkoxy, Halogenalkylthio mit jeweils 1 bis 4 Kohlenstoffatomen und 1 bis 9

gleichen oder verschiedenen Halogenatomen,
außerdem für jeweils gegebenenfalls im Aryl- bzw. Heterocyclylteil einfach bis fünffach, gleich oder verschieden substituiertes Arylalkyl, Aryl, Heterocyclylalkyl oder Heterocyclyl mit jeweils gegebenenfalls 6 bis 10 Kohlenstoffatomen im Arylteil bzw. 2 bis 9 Kohlenstoffatomen und 1 bis 3 gleichen oder verschiedenen Heteroatomen - insbesondere Stickstoff, Sauerstoff und/oder Schwefel - im Heterocyclylteil und gegebenenfalls 1 bis 4 Kohlenstoffatomen im geradkettigen oder verzweigten Alkylteil steht, wobei als Aryl- bzw. Heterocyclylsubstituenten jeweils die bei $R^1$ genannten infrage kommen, oder zusammen einen Cycloalkylring mit 3 bis 6 Kohlenstoffatomen bilden,

n für eine Zahl 0, 1, 2 oder 3 steht,
$X^1$ für Sauerstoff oder Schwefel steht,
$X^2$ für Sauerstoff oder Schwefel steht und
$X^3$ für Sauerstoff oder Schwefel steht,

wobei jedoch mindestens einer der Substituenten $X^1$, $X^2$ und/oder $X^3$ für Schwefel steht.

**4.** Verbindungen der Formel (I) gemäß Anspruch 1, bei welchen

$R^1$ für jeweils geradkettiges oder verzweigtes Alkyl mit 2 bis 5 Kohlenstoffatomen, Alkenyl mit 2 bis 4 Kohlenstoffatomen, Alkinyl mit 2 bis 4 Kohlenstoffatomen, Halogenalkyl mit 1 oder 2 Kohlenstoffatomen und 1 bis 5 gleichen oder verschiedenen Halogenatomen, Halogenalkenyl mit 2 bis 3 Kohlenstoffatomen und 1 bis 5 gleichen oder verschiedenen Halogenatomen, Halogenalkinyl mit 2 bis 3 Kohlenstoffatomen und 1 bis 5 gleichen oder verschiedenen Halogenatomen, Cycloalkyl mit 3 bis 6 Kohlenstoffatomen, Cycloalkenyl mit 3 bis 6 Kohlenstoffatomen, oder für jeweils gegebenenfalls im Phenylteil einfach bis dreifach, gleich oder verschieden substituiertes Phenylalkyl oder Phenyl mit gegebenenfalls 1 bis 4 Kohlenstoffatomen im geradkettigen oder verzweigten Alkylteil steht, wobei als Phenylsubstituenten jeweils infrage kommen:
Halogen, Cyano, Nitro, Hydroxy, Formyl, Hydroxycarbonyl, Aminocarbonyl, Aminosulfonyl, jeweils geradkettiges oder verzweigtes Alkyl, Alkoxy, Alkoxyalkyl, Alkylthio, Alkylsulfinyl, Alkylsulfonyl, Alkoxysulfonyl, Alkylsulfonyloxy oder Dialkylamino mit jeweils 1 bis 3 Kohlenstoffatomen in den einzelnen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 oder 2 Kohlenstoffatomen und 1 bis 5 gleichen oder verschiedenen Halogenatomen, jeweils geradkettiges oder verzweigtes Alkylcarbonyl, Alkylcarbonyloxy, Alkoxycarbonyl, N-Alkylaminocarbonyl, N,N-Dialkylaminocarbonyl, Alkylcarbonylamino, Alkylcarbonylaminoalkyl oder Alkoximinoalkyl mit jeweils 1 bis 3 Kohlenstoffatomen in den einzelnen Alkylteilen sowie jeweils gegebenenfalls im Phenylteil einfach oder mehrfach, gleich oder verschieden durch Halogen und/oder geradkettiges oder verzweigtes Alkyl mit 1 bis 3 Kohlenstoffatomen substituiertes Phenyl, Phenoxy, Phenylcarbonyl, Phenoxycarbonyl oder Phenylcarbonyloxy,

$R^2$ für Wasserstoff, für geradkettiges oder verzweigtes Alkyl mit 1 bis 4 Kohlenstoffatomen oder für Cycloalkyl mit 3 bis 6 Kohlenstoffatomen steht,

$R^3$ für Wasserstoff, für geradkettiges oder verzweigtes Alkyl mit 1 bis 4 Kohlenstoffatomen oder für Cycloalkyl mit 3 bis 6 Kohlenstoffatomen steht und

$R^4$ für geradkettiges oder verzweigtes Alkyl mit 1 bis 4 Kohlenstoffatomen oder für Cycloalkyl mit 3 bis 6 Kohlenstoffatomen steht oder

$R^3$ und $R^4$ gemeinsam mit dem Kohlenstoffatom, an welches sie gebunden sind, für einen 3- bis 6-gliedrigen Cycloalkylring stehen,

$R^5$ für jeweils gegebenenfalls einfach bis dreifach, gleich oder verschieden substituiertes Cycloalkyl, Cycloalkylalkyl, Cycloalkenylalkyl oder Cycloalkenyl mit jeweils 3 bis 6 Kohlenstoffatomen steht, wobei als Cycloalkyl- bzw. Cycloalkenylsubstituenten jeweils infrage kommen:
Halogen, Hydroxy, jeweils geradkettiges oder verzweigtes Alkyl, Alkandiyl, Alkoxy, Alkoxyalkyl, Alkylthio oder Dialkylamino mit jeweils 1 bis 3 Kohlenstoffatomen in den einzelnen Alkylteilen oder jeweils geradkettiges oder verzweigtes Halogenalkyl, Halogenalkoxy, Halogenalkylthio mit jeweils 1 oder 2 Kohlenstoffatomen und 1 bis 5 gleichen oder verschiedenen Halogenatomen,

außerdem für jeweils gegebenenfalls einfach bis dreifach, gleich oder verschieden substituiertes Phenyl oder Phenylalkyl oder Heterocyclyl oder Heterocyclylalkyl mit 2 bis 9 Kohlenstoffatomen und 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils die bei $R^1$ genannten infrage kommen,

$R^7$, $R^6$ unabhängig voneinander für Wasserstoff, für jeweils geradkettiges oder verzweigtes Alkyl mit 1 bis 4 Kohlenstoffatomen, Cyanalkyl mit 2 bis 3 Kohlenstoffatomen, Alkenyl mit 2 bis 4 Kohlenstoffatomen, Alkinyl mit 2 bis 4 Kohlenstoffatomen oder für jeweils gegebenenfalls im Cycloalkyl-bzw. Cycloalkenylteil einfach bis dreifach, gleich oder verschieden substituiertes Cycloalkyl, Cycloalkylalkyl, Cycloalkenyl oder Cycloalkenylalkyl mit jeweils 3 bis 6 Kohlenstoffatomen im Cycloalkyl- bzw. Cycloalkenylteil und gegebenenfalls 1 bis 3 Kohlenstoffatomen im geradkettigen oder verzweigten Alkylteil steht, wobei als Cycloalkyl- bzw. Cycloalkenylsubstituenten jeweils infrage kommen:
Halogen, Hydroxy, jeweils geradkettiges oder verzweigtes Alkyl, Alkandiyl, Alkoxy, Alkoxyalkyl, Alkylthio oder Dialkylamino mit jeweils 1 bis 3 Kohlenstoffatomen in den einzelnen Alkylteilen oder jeweils geradkettiges oder verzweigtes Halogenalkyl, Halogenalkoxy, Halogenalkylthio mit jeweils 1 oder 2 Kohlenstoffatomen und 1 bis 5 gleichen oder verschiedenen Halogenatomen,
außerdem für jeweils gegebenenfalls im Phenyl- bzw. Heterocyclylteil einfach bis dreifach, gleich oder verschieden substituiertes Phenylalkyl, Phenyl, Heterocyclylalkyl oder Heterocyclyl mit jeweils gegebenenfalls 2 bis 9 Kohlenstoffatomen und 1 bis 3 gleichen oder verschiedenen Heteroatomen - insbesondere Stickstoff, Sauerstoff und/oder Schwefel - im Heterocyclylteil und gegebenenfalls 1 bis 3 Kohlenstoffatomen im geradkettigen oder verzweigten Alkylteil steht, wobei als Aryl- bzw. Heterocyclylsubstituenten jeweils die bei $R^1$ genannten infrage kommen, oder zusammen einen Cycloalkylring mit 3 bis 5 Kohlenstoffatomen bilden,

n für eine Zahl 1 oder 2 steht,

$X^1$ für Sauerstoff oder Schwefel steht,

$X^2$ für Sauerstoff oder Schwefel steht und

$X^3$ für Sauerstoff oder Schwefel steht,

wobei jedoch mindestens einer der Substituenten $X^1$, $X^2$ und/oder $X^3$ für Schwefel steht.

**5.** Schädlingsbekämpfungsmittel, gekennzeichnet durch einen Gehalt an mindestens einem Aminosäureamid der Formel (I) nach Anspruch 1.

**6.** Verfahren zur Bekämpfung von Schädlingen, dadurch gekennzeichnet, daß man Aminosäureamide der Formel (I) nach Anspruch 1 auf Schädlinge und/oder ihren Lebensraum einwirken läßt.

**7.** Verfahren zur Herstellung von Aminosäureamiden der allgemeinen Formel (I),

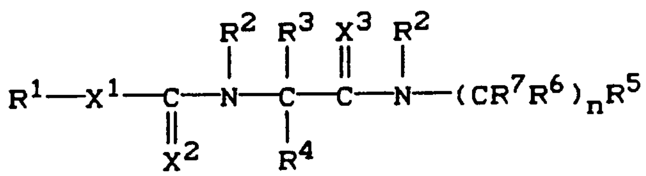

(I)

in welcher
n, $R^1$, $R^2$, $R^3$, $R^4$, $R^5$, $R^6$, $R^7$, $X^1$, $X^2$ und $X^3$ die in Anspruch 1 angegebene Bedeutung haben, dadurch gekennzeichnet, daß man substituierte Aminosäuren der Formel (II)

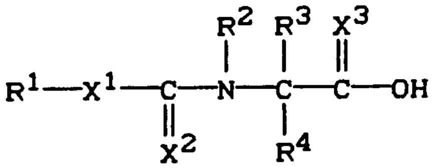

(II)

in welcher
$R^1$, $R^2$, $R^3$, $R^4$, $X^1$, $X^2$ und $X^3$ die oben angegebene Bedeutung haben,
mit Aminen der Formel (III),

$R^2HN-(CR^7R^6)_nR^5$ (III)

in welcher
n, $R^2$, $R^5$, $R^6$ und $R^7$ die oben angegebene Bedeutung haben,
gegebenenfalls in Gegenwart eines Verdünnungsmittels und gegebenenfalls in Gegenwart eines Reaktionshilfsmittels umsetzt.

**8.** Verwendung von Aminosäureamiden der Formel (I) nach den Ansprüchen 1 bis 7 zur Bekämpfung von Schädlingen.

**9.** Verfahren zur Herstellung von Schädlingsbekämpfungsmitteln, dadurch gekennzeichnet, daß man Aminosäureamide der Formel (I) nach den Ansprüchen 1 bis 7 mit Streckmitteln und/oder oberflächenaktiven Mitteln vermischt.

**Claims**

**1.** Amino acid amides of the formula (I)

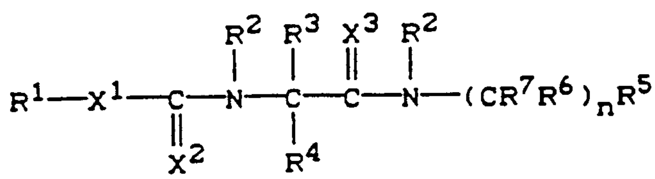

(I)

in which

| | |
|---|---|
| $R^1$ | represents alkyl, alkenyl, alkinyl, halogenoalkyl, halogenoalkenyl, halogenoalkinyl, cycloalkyl, cycloalkenyl, or in each case optionally substituted arylalkyl, aryl, heteroarylalkyl or heteroaryl, |
| $R^2$ | represents hydrogen, alkyl or cycloalkyl, |
| $R^3$ | represents hydrogen, alkyl or cycloalkyl and |
| $R^4$ | represents alkyl, cycloalkyl, or |
| $R^3$ and $R^4$ | together with the carbon atom to which they are bonded represent a cycloalkyl ring, |
| $R^5$ | represents in each case optionally substituted cycloalkyl, cycloalkenyl, aryl, heterocyclyl, cycloalkylalkyl, cycloalkenylalkyl, arylalkyl or heterocyclylalkyl, |
| $R^7$, $R^6$ | independently of one another represent hydrogen or alkyl, alkenyl, alkinyl, cycloalkyl, cycloalkenyl, aryl, heterocyclyl, cycloalkylalkyl, cycloalkenylalkyl, arylalkyl or heterocyclylalkyl, each of which is optionally substituted, or together form a cycloalkyl ring, |
| n | represents a number 0, 1, 2 or 3, |
| $X^1$ | represents oxygen or sulphur, |
| $X^2$ | represents oxygen or sulphur, and |
| $X^3$ | represents oxygen or sulphur, |

but where at least one of the substituents $X^1$, $X^2$ and/or $X^3$ represents sulphur.

**2.** Amino acid amides of the formula (I) according to Claim 1, in which

$R^1$ represents in each case straight-chain or branched alkyl having 1 to 8 carbon atoms, alkenyl having 2 to 8 carbon atoms, alkinyl having 2 to 8 carbon atoms, halogenoalkyl having 1 to 6 carbon atoms and 1 to 13 identical or different halogen atoms, halogenoalkenyl having 2 to 6 carbon atoms and 1 to 11 identical or different halogen atoms, halogenoalkinyl having 2 to 6 carbon atoms and 1 to 11 identical or different halogen atoms, cycloalkyl having 3 to 8 carbon atoms, cycloalkenyl having 3 to 8 carbon atoms, or represents arylalkyl, aryl, heteroarylalkyl or heteroaryl, each of which has, if appropriate, 6 to 10 carbon atoms in the aryl moiety, or 2 to 9 carbon atoms and 1 to 4 identical or different hetero atoms - in particular nitrogen, oxygen and/or sulphur - in the heteroaryl moiety and, if appropriate, 1 to 6 carbon atoms in the straight-chain or branched alkyl moiety, and each of which is optionally monosubstituted or polysubstituted in the aryl moiety, or heteroaryl moiety, by identical or different substituents, suitable aryl or heteroaryl substituents in each case being:
halogen, cyano, nitro, hydroxyl, formyl, hydroxycarbonyl, aminocarbonyl, aminosulphonyl, in each case straight-chain or branched alkyl, alkoxy, alkoxyalkyl, alkylthio, alkylsulphinyl, alkylsulphonyl, alkoxysulphonyl, alkylsulphonyloxy or dialkylamino, each of which has 1 to 4 carbon atoms in the individual alkyl moieties, in each case straight-chain or branched halogenoalkyl, halogenoalkoxy, halogenoalkylthio, halogenoalkylsulphinyl or halogenoalkylsulphonyl, each of which has 1 to 4 carbon atoms and 1 to 9 identical or different halogen atoms, in each case straight-chain or branched alkylcarbonyl, alkylcarbonyloxy, alkoxycarbonyl, N-alkylaminocarbonyl, N,N-dialkylaminocarbonyl, alkylcarbonylamino, alkylcarbonylaminoalkyl or alkoximinoalkyl, each of which has 1 to 4 carbon atoms in the individual alkyl moieties, and phenyl, phenoxy, phenylcarbonyl, phenoxycarbonyl or phenylcarbonyloxy, each of which is optionally monosubstituted or polysubstituted in the phenyl moiety by identical or different substituents from the series comprising halogen and/or straight-chain or branched alkyl having 1 to 4 carbon atoms,

$R^2$ represents hydrogen, straight-chain or branched alkyl having 1 to 8 carbon atoms or cycloalkyl having 3 to 8 carbon atoms,

$R^3$ represents hydrogen, straight-chain or branched alkyl having 1 to 8 carbon atoms or cycloalkyl having 3 to 8 carbon atoms and

$R^4$ represents straight-chain or branched alkyl having 1 to 8 carbon atoms or cycloalkyl having 3 to 8 carbon atoms, or

$R^3$ and $R^4$ together with the carbon atom to which they are bonded represent a 3- to 8-membered cycloalkyl ring,

$R^5$ represents cycloalkyl or cycloalkylalkyl or cycloalkenyl or cycloalkenylalkyl, each of which has 3 to 8 carbon atoms and each of which is optionally monosubstituted or polysubstituted by identical or different substituents, suitable substituents in each case being:
halogen, hydroxyl, in each case straight-chain or branched alkyl, alkanediyl, alkoxy, alkoxyalkyl, alkylthio or dialkylamino, each of which has 1 to 6 carbon atoms in the individual alkyl moieties, or in each case straight-chain or branched halogenoalkyl, halogenoalkoxy, halogenoalkylthio, each of which has 1 to 4 carbon atoms and 1 to 9 identical or different halogen atoms,
moreover aryl or arylalkyl having 6 to 10 carbon atoms or heterocyclyl or heterocyclylalkyl having 2 to 9 carbon atoms and 1 to 4 identical or different hetero atoms - in particular nitrogen, oxygen and/or sulphur, each of these aryl or heterocyclyl radicals optionally being monosubstituted or polysubstituted by identical or different substituents, suitable aryl or heterocyclyl substituents in each case being those mentioned in the case of $R^1$,

$R^7$, $R^6$ independently of one another represent hydrogen, in each case straight-chain or branched alkyl having 1 to 8 carbon atoms, cyanoalkyl having 2 to 9 carbon atoms, alkenyl having 2 to 8 carbon atoms, alkinyl having 2 to 8 carbon atoms, or cycloalkyl, cycloalkylalkyl, cycloalkenyl or cycloalkenylalkyl, each of which has 3 to 8 carbon atoms in the cycloalkyl or cycloalkenyl moiety and, if appropriate, 1 to 6 carbon atoms in the straight-chain or branched alkyl moiety, and each of which is optionally monosubstituted or polysubstituted in the cycloalkyl or cycloalkenyl moiety by identical or different substituents, suitable cycloalkyl or cycloalkenyl substituents in each case being:

halogen, hydroxyl, in each case straight-chain or branched alkyl, alkanediyl, alkoxy, alkoxyalkyl, alkylthio or dialkylamino, each of which has 1 to 6 carbon atoms in the individual alkyl moieties, or in each case straight-chain or branched halogenoalkyl, halogenoalkoxy, halogenoalkylthio, each of which has 1 to 4 carbon atoms and 1 to 9 identical or different halogen atoms,

moreover arylalkyl, aryl, heterocyclylalkyl or heterocyclyl, each of which has, if appropriate, 6 to 10 carbon atoms in the aryl moiety or 2 to 9 carbon atoms and 1 to 4 identical or different hetero atoms - in particular nitrogen, oxygen and/or sulphur - in the heterocyclyl moiety and, where appropriate, 1 to 6 carbon atoms in the straight-chain or branched alkyl moiety, and each of which is optionally monosubstituted or polysubstituted in the aryl or heterocyclyl moiety by identical or different substituents, suitable aryl or heterocyclyl substituents in each case being those mentioned in the case of $R^1$, or together form a cycloalkyl ring having 3 to 7 carbon atoms,

n represents a number 0, 1, 2 or 3,

$X^1$ represents oxygen or sulphur,

$X^2$ represents oxygen or sulphur and

$X^3$ represents oxygen or sulphur,

but where at least one of the substituents $X^1$, $X^2$ and/or $X^3$ represents sulphur.

**3.** Compounds of the formula (I) according to Claim 1, in which

$R^1$ represents in each case straight-chain or branched alkyl having 1 to 6 carbon atoms, alkenyl having 2 to 6 carbon atoms, alkinyl having 2 to 6 carbon atoms, halogenoalkyl having 1 to 4 carbon atoms and 1 to 9 identical or different halogen atoms, halogenoalkenyl having 2 to 4 carbon atoms and 1 to 7 identical or different halogen atoms, halogenoalkinyl having 2 to 4 carbon atoms and 1 to 7 identical or different halogen atoms, cycloalkyl having 3 to 7 carbon atoms, cycloalkenyl having 3 to 7 carbon atoms, or represents arylalkyl, aryl, heteroarylalkyl or heteroaryl, each of which has, where appropriate, 6 to 10 carbon atoms in the aryl moiety, or 2 to 9 carbon atoms and 1 to 3 identical or different hetero atoms - in particular nitrogen, oxygen and/or sulphur - in the heteroaryl moiety and, where appropriate, 1 to 4 carbon atoms in the straight-chain or branched alkyl moiety, and each of which is optionally monosubstituted to pentasubstituted in the aryl or heteroaryl moiety by identical or different substituents, suitable aryl or heteroaryl substituents in each case being:

halogen, cyano, nitro, hydroxyl, formyl, hydroxycarbonyl, aminocarbonyl, aminosulphonyl, in each case straight-chain or branched alkyl, alkoxy, alkoxyalkyl, alkylthio, alkylsulphinyl, alkylsulphonyl, alkoxysulphonyl, alkylsulphonyloxy or dialkylamino, each of which has 1 to 4 carbon atoms in the individual alkyl moieties, in each case straight-chain or branched halogenoalkyl, halogenoalkoxy, halogenoalkylthio, halogenoalkylsulphinyl or halogenoalkylsulphonyl, each of which has 1 to 4 carbon atoms and 1 to 9 identical or different halogen atoms, in each case straight-chain or branched alkylcarbonyl, alkylcarbonyloxy, alkoxycarbonyl, N-alkylaminocarbonyl, N,N-dialkylaminocarbonyl, alkylcarbonylamino, alkylcarbonylaminoalkyl or alkoximinoalkyl, each of which has 1 to 4 carbon atoms in the individual alkyl moieties, and phenyl, phenoxy, phenylcarbonyl, phenoxycarbonyl or phenylcarbonyloxy, each of which is optionally monosubstituted or polysubstituted in the phenyl moiety by identical or different substituents from the series comprising halogen and/or straight-chain or branched alkyl having 1 to 4 carbon atoms,

$R^2$ represents hydrogen, straight-chain or branched alkyl having 1 to 6 carbon atoms or cycloalkyl having 3 to 7 carbon atoms,

$R^3$ represents hydrogen, straight-chain or branched alkyl having 1 to 6 carbon atoms or cycloalkyl having 3 to 7 carbon atoms and

$R^4$ represents straight-chain or branched alkyl having 1 to 6 carbon atoms or cycloalkyl having 3 to 6 carbon atoms, or

$R^3$ and $R^4$ together with the carbon atom to which they are bonded represent a 3- to 7-membered cycloalkyl ring,

$R^5$ represents cycloalkyl, cycloalkylalkyl, cycloalkenylalkyl or cycloalkenyl, each of which

has 3 to 7 carbon atoms and each of which is optionally monosubstituted to pentasubstituted by identical or different substituents, suitable substituents in each case being:

halogen, hydroxyl, in each case straight-chain or branched alkyl, alkanediyl, alkoxy, alkoxyalkyl, alkylthio or dialkylamino, each of which has 1 to 4 carbon atoms in the individual alkyl moieties, or in each case straight-chain or branched halogenoalkyl, halogenoalkoxy, halogenoalkylthio, each of which has 1 to 4 carbon atoms and 1 to 9 identical or different halogen atoms,

moreover represents aryl or arylalkyl having 6 to 10 carbon atoms or heterocyclyl or heterocyclylalkyl having 2 to 9 carbon atoms and 1 to 3 identical or different hetero atoms - in particular nitrogen, oxygen and/or sulphur, each of these aryl or heterocyclyl radicals optionally being monosubstituted to pentasubstituted by identical or different substituents, suitable substituents in each case being those mentioned in the case of $R^1$,

$R^7$, $R^6$ independently of one another represent hydrogen, in each case straight-chain or branched alkyl having 1 to 6 carbon atoms, cyanoalkyl having 2 to 5 carbon atoms, alkenyl having 2 to 6 carbon atoms, alkinyl having 2 to 6 carbon atoms, or cycloalkyl, cycloalkylalkyl, cycloalkenyl or cycloalkenylalkyl, each of which has 3 to 7 carbon atoms in the cycloalkyl or cycloalkenyl moiety and, where appropriate, 1 to 4 carbon atoms in the straight-chain or branched alkyl moiety, and each of which is optionally monosubstituted to pentasubstituted in the cycloalkyl or cycloalkenyl moiety by identical or different substituents, suitable cycloalkyl or cycloalkenyl substituents in each case being:

halogen, hydroxyl, in each case straight-chain or branched alkyl, alkanediyl, alkoxy, alkoxyalkyl, alkylthio or dialkylamino, each of which has 1 to 4 carbon atoms in the individual alkyl moieties, or in each case straight-chain or branched halogenoalkyl, halogenoalkoxy, halogenoalkylthio, each of which has 1 to 4 carbon atoms and 1 to 9 identical or different halogen atoms,

moreover represents arylalkyl, aryl, heterocyclylalkyl or heterocyclyl, each of which has, if appropriate, 6 to 10 carbon atoms in the aryl moiety or 2 to 9 carbon atoms and 1 to 3 identical or different hetero atoms - in particular nitrogen, oxygen and/or sulphur - in the heterocyclyl moiety and, where appropriate, 1 to 4 carbon atoms in the straight-chain or branched alkyl moiety, and each of which is optionally monosubstituted to pentasubstituted in the aryl or heterocyclyl moiety by identical or different substituents, suitable aryl or heterocyclyl substituents in each case being those mentioned in the case of $R^1$, or together form a cycloalkyl ring having 3 to 6 carbon atoms,

n represents a number 0, 1, 2 or 3,

$X^1$ represents oxygen or sulphur,

$X^2$ represents oxygen or sulphur and

$X^3$ represents oxygen or sulphur,

but where at least one of the substituents $X^1$, $X^2$ and/or $X^3$ represents sulphur.

**4.** Compounds of the formula (I) according to Claim 1, in which

$R^1$ represents in each case straight-chain or branched alkyl having 2 to 5 carbon atoms, alkenyl having 2 to 4 carbon atoms, alkinyl having 2 to 4 carbon atoms, halogenoalkyl having 1 or 2 carbon atoms and 1 to 5 identical or different halogen atoms, halogenoalkenyl having 2 to 3 carbon atoms and 1 to 5 identical or different halogen atoms, halogenoalkinyl having 2 to 3 carbon atoms and 1 to 5 identical or different halogen atoms, cycloalkyl having 3 to 6 carbon atoms, cycloalkenyl having 3 to 6 carbon atoms, or represents phenylalkyl or phenyl, each of which has, where appropriate, 1 to 4 carbon atoms in the straight-chain or branched alkyl moiety, and each of which is optionally monosubstituted to trisubstituted in the phenyl moiety by identical or different substituents, suitable phenyl substituents in each case being:

halogen, cyano, nitro, hydroxyl, formyl, hydroxycarbonyl, aminocarbonyl, aminosulphonyl, in each case straight-chain or branched alkyl, alkoxy, alkoxyalkyl, alkylthio, alkylsulphinyl, alkylsulphonyl, alkoxysulphonyl, alkylsulphonyloxy or dialkylamino, each of which has 1 to 3 carbon atoms in the individual alkyl moieties, in each case

straight-chain or branched halogenoalkyl, halogenoalkoxy, halogenoalkylthio, halogenoalkylsulphinyl or halogenoalkylsulphonyl, each of which has 1 or 2 carbon atoms and 1 to 5 identical or different halogen atoms, in each case straight-chain or branched alkylcarbonyl, alkylcarbonyloxy, alkoxycarbonyl, N-alkylaminocarbonyl, N,N-dialkylaminocarbonyl, alkylcarbonylamino, alkylcarbonylaminoalkyl or alkoximinoalkyl, each of which has 1 to 3 carbon atoms in the individual alkyl moieties, and phenyl, phenoxy, phenylcarbonyl, phenoxycarbonyl or phenylcarbonyloxy, each of which is optionally monosubstituted or polysubstituted in the phenyl moiety by identical or different substituents from the series comprising halogen and/or straight-chain or branched alkyl having 1 to 3 carbon atoms,

$R^2$ represents hydrogen, straight-chain or branched alkyl having 1 to 4 carbon atoms or cycloalkyl having 3 to 6 carbon atoms,

$R^3$ represents hydrogen, straight-chain or branched alkyl having 1 to 4 carbon atoms or cycloalkyl having 3 to 6 carbon atoms and

$R^4$ represents straight-chain or branched alkyl having 1 to 4 carbon atoms or cycloalkyl having 3 to 6 carbon atoms, or

$R^3$ and $R^4$ together with the carbon atom to which they are bonded represent a 3- to 6-membered cycloalkyl ring,

$R^5$ represents cycloalkyl, cycloalkylalkyl, cycloalkenylalkyl or cycloalkenyl, each of which has 3 to 6 carbon atoms and each of which is optionally monosubstituted to trisubstituted by identical or different substituents, suitable cycloalkyl or cycloalkenyl substituents in each case being:

halogen, hydroxyl, in each case straight-chain or branched alkyl, alkanediyl, alkoxy, alkoxyalkyl, alkylthio or dialkylamino, each of which has 1 to 3 carbon atoms in the individual alkyl moieties, or in each case straight-chain or branched halogenoalkyl, halogenoalkoxy, halogenoalkylthio, each of which has 1 or 2 carbon atoms and 1 to 5 identical or different halogen atoms,

moreover phenyl or phenylalkyl or heterocyclyl or heterocyclylalkyl having 2 to 9 carbon atoms and 1 to 3 identical or different hetero atoms - in particular nitrogen, oxygen and/or sulphur, each of these phenyl or heterocyclyl radicals optionally being monosubstituted to trisubstituted by identical or different substituents, suitable phenyl or heterocyclyl substituents in each case being those mentioned in the case of $R^1$,

$R^7$, $R^6$ independently of one another represent hydrogen, in each case straight-chain or branched alkyl having 1 to 4 carbon atoms, cyanoalkyl having 2 to 3 carbon atoms, alkenyl having 2 to 4 carbon atoms, alkinyl having 2 to 4 carbon atoms, or cycloalkyl, cycloalkylalkyl, cycloalkenyl or cycloalkenylalkyl, each of which has 3 to 6 carbon atoms in the cycloalkyl or cycloalkenyl moiety and, where appropriate, 1 to 3 carbon atoms in the straight-chain or branched alkyl moiety, and each of which is optionally monosubstituted to trisubstituted in the cycloalkyl or cycloalkenyl moiety by identical or different substituents, suitable cycloalkyl or cycloalkenyl substituents in each case being:

halogen, hydroxyl, in each case straight-chain or branched alkyl, alkanediyl, alkoxy, alkoxyalkyl, alkylthio or dialkylamino, each of which has 1 to 3 carbon atoms in the individual alkyl moieties, or in each case straight-chain or branched halogenoalkyl, halogenoalkoxy, halogenoalkylthio, each of which has 1 or 2 carbon atoms and 1 to 5 identical or different halogen atoms,

moreover represents phenylalkyl, phenyl, heterocyclylalkyl or heterocyclyl, each of which has, where appropriate, 2 to 9 carbon atoms and 1 to 3 identical or different hetero atoms - in particular nitrogen, oxygen and/or sulphur - in the heterocyclyl moiety and, where appropriate, 1 to 3 carbon atoms in the straight-chain or branched alkyl moiety, and each of which is optionally monosubstituted to trisubstituted in the phenyl or heterocyclyl moiety by identical or different substituents, suitable aryl or heterocyclyl substituents in each case being those mentioned in the case of $R^1$, or together form a cycloalkyl ring having 3 to 5 carbon atoms,

n represents a number 1 or 2,

$X^1$ represents oxygen or sulphur,

$X^2$ represents oxygen or sulphur and

$X^3$ represents oxygen or sulphur,

but where at least one of the substituents $X^1$, $X^2$ and/or $X^3$ represents sulphur.

5. Pesticides, characterised in that they contain at least one amino acid amide of the formula (I) according to Claim 1.

6. Method of combating pests, characterised in that amino acid amides of the formula (I) according to Claim 1 are allowed to act on pests and/or their environment.

7. Process for the preparation of amino acid amides of the general formula (I)

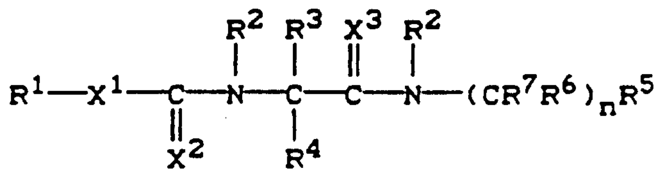

(I)

in which
n, $R^1$, $R^2$, $R^3$, $R^4$, $R^5$, $R^6$, $R^7$, $X^1$, $X^2$ and $X^3$ have the meaning given in Claim 1,
characterised in that substituted amino acids of the formula (II)

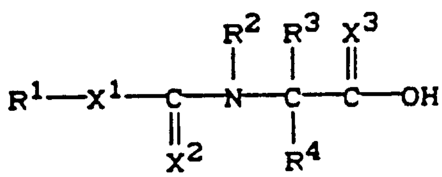

(II)

in which
$R^1$, $R^2$, $R^3$, $R^4$, $X^1$, $X^2$ and $X^3$ have the abovementioned meaning,
are reacted with amines of the formula (III)

$R^2HN-(CR^7R^6)_nR^5$ (III)

in which
n, $R^2$, $R^5$, $R^6$ and $R^7$ have the abovementioned meaning, if appropriate in the presence of a diluent and if appropriate in the presence of a reaction auxiliary.

8. Use of amino acid amides of the formula (I) according to Claims 1 to 7 for combating pests.

9. Process for the preparation of pesticides, characterised in that amino acid amides of the formula (I) according to Claims 1 to 7 are mixed with extenders and/or surface-active agents.

**Revendications**

1. Amides d'aminoacides de formule (I),

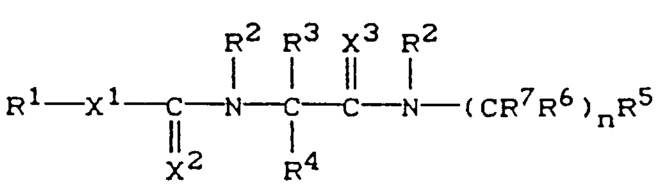

(I)

dans laquelle

$R^1$ est un groupe alkyle, alcényle, alcynyle, halogénalkyle, halogénalcényle, halogénalcynyle, cycloalkyle, cycloalcényle ou un groupe arylalkyle, aryle, hétéroarylalkyle ou hétéroaryle dont chacun est éventuellement substitué,
$R^2$ représente de l'hydrogène, un groupe alkyle ou cycloalkyle,
$R^3$ représente de l'hydrogène, un groupe alkyle ou cyclalkyle et
$R^4$ est un groupe alkyle ou cycloalkyle, ou bien
$R^3$ et $R^4$ forment un noyau cycloalkyle conjointement avec l'atome de carbone auquel ils sont liés,
$R^5$ représente un groupe cycloalkyle, cycloalcényle, aryle, hétérocyclyle, cycloalkylalkyle, cycloalcénylalkyle, arylalkyle ou hétérocyclylalkyle, chacun étant éventuellement substitué,
$R^7$, $R^6$ représentent indépendamment l'un de l'autre de l'hydrogène ou un groupe alkyle, alcényle, alcynyle, cycloalkyle, cycloalcényle, aryle, hétérocyclyle, cycloalkylalkyle, cycloalcénylalkyle, arylalkyle ou hétérocyclylalkyle, chacun étant éventuellement substitué, ou forment ensemble un noyau cycloalkyle,
n représente le noire 0, 1, 2 ou 3,
$X^1$ est de l'oxygène ou du soufre,
$X^2$ est de l'oxygène ou du soufre et
$X^3$ est un oxygène ou du soufre,

l'un au moins des substituants $X^1$, $X^2$ et/ou $X^3$ représentant toutefois du soufre.

**2.** Amides d'aminoacides de formule (I) suivant la revendication 1, dans lesquels

$R^1$ représente un groupe, linéaire ou ramifié dans chaque cas, alkyle ayant 1 à 8 atomes de carbone, alcényle ayant 2 à 8 atomes de carbone, alcynyle ayant 2 à 8 atomes de carbone, halogénalkyle ayant 1 à 6 atomes de carbone et 1 à 13 atomes d'halogènes identiques ou différents, halogénalcényle ayant 2 à 6 atomes de carbone et 1 à 11 atomes d'halogènes identiques ou différents, halogénalcynyle ayant 2 à 6 atomes de carbone et 1 à 11 atomes d'halogènes identiques ou différents, un groupe cycloalkyle de 3 à 8 atomes de carbone, cycloalcényle de 3 à 8 atomes de carbone, ou un groupe arylalkyle, aryle, hétéroarylalkyle ou hétéroaryle portant chacun le cas échéant un ou plusieurs substituants identiques ou différents dans la partie aryle ou hétéroaryle et ayant chacun le cas échéant 6 à 10 atomes de carbone dans la partie aryle, ou 2 à 9 atomes de carbone et 1 à 4 hétéroatomes identiques ou différents, notamment des atomes d'azote, d'oxygène et/ou de soufre, dans la partie hétéroaryle et le cas échéant 1 à 6 atomes de carbone dans la partie alkyle linéaire ou ramifiée, en considérant dans chaque cas comme substituants de la partie aryle ou hétéroaryle :
un radical halogéno, cyano, nitro, hydrocy, formyle, hydroxycarbonyle, aminocarbonyle, aminosulfonyle, un radical alkyle, alkoxy, alkoxyalkyle, alkylthio, alkylsulfinyle, alkoxysulfonyle, alkylsulfonyle, alkylsulfonyloxy ou dialkylamino linéaire ou ramifié dans chaque cas, ayant chacun 1 à 4 atomes de carbone dans les parties alkyle individuelles, un radical halogénalkyle, halogénalkoxy, halogénalkylthio, halogénalkylsulfinyle ou halogénalkylsulfonyle linéaire ou ramifié dans chaque cas, ayant chacun 1 à 4 atomes de carbone et 1 à 9 atomes d'halogènes identiques ou différents, un radical alkylcarbonyle, alkylcarbonyloxy, alkoxycarbonyle, N-alkylaminocarbonyle, N,N-dialkylaminocarbonyle, alkylcarbonylamino, alkylcarbonylaminoalkyle ou alkoximinoalkyle linéaire ou ramifié dans chaque cas, ayant chacun 1 à 4 atomes de carbone dans les parties alkyle individuelles ainsi qu'un radical phényle, phénoxy, phénylcarbonyle, phénoxycarbonyle ou phénylcarbonyloxy portant chacun le cas échéant dans la partie phényle un ou plusieurs substituants, identiques ou différents, halogéno et/ou alkyle linéaire ou ramifié ayant 1 à 4 atomes de carbone,
$R^2$ représente de l'hydrogène, un groupe alkyle linéaire ou ramifié ayant 1 à 8 atomes atomes de carbone ou un groupe cycloalkyle ayant 3 à 8 atomes de carbone,
$R^3$ est de l'hydrogène, un groupe alkyle linéaire ou ramifié ayant 1 à 8 atomes de carbone ou un groupe cycloalkyle ayant 3 à 8 atomes de carbone et
$R^4$ est un groupe alkyle linéaire ou ramifié ayant 1 à 8 atomes de carbone ou un groupe cycloalkyle ayant 3 à 8 atomes de carbone, ou bien
$R^3$ et $R^4$ forment conjointement avec l'atome de carbone auquel ils sont liés un noyau cyclalkyle de 3 à 8 chaînons,

$R^5$ est un groupe cycloalkyle ou cycloalkylalkyle ou cycloalcényle ou cycloalcénylalkyle portant chacun le cas échéant un ou plusieurs substituants identiques ou différents et ayant chacun 1 à 8 atomes de carbone, en considérant dans chaque cas comme substituants : un radical halogéno, hydroxy, un radical alkyle, alcanediyle, alkoxy, alkoxyalkyle, alkylthio ou dialkylamino linéaire ou ramifié dans chaque cas, ayant chacun 1 à 6 atomes de carbone dans les parties alkyle individuelles, ou un radical halogénalkyle, halogénalkoxy, halogénalkylthio linéaire ou ramifié dans chaque cas ayant chacun 1 à 4 atomes de carbone et 1 à 9 atomes d'halogènes identiques ou différents,
en outre un radical aryle ou arylalkyle de 6 à 10 atomes de carbone portant chacun le cas échéant un ou plusieurs substituants identiques ou différents ou un radical hétérocyclyle ou hétérocyclylalkyle ayant 2 à 9 atomes de carbone et 1 à 4 hétéroatomes identiques ou différents - notamment azote, oxygène et/ou soufre - en considérant dans chaque cas comme substituants de la partie aryle ou hétérocyclyle les substituants mentionnés pour $R^1$,

$R^7$, $R^6$ représentent indépendamment l'un de l'autre un groupe, linéaire ou ramifié, alkyle ayant 1 à 8 atomes de carbone, cyanalkyle ayant 2 à 9 atomes de carbone, alcényle ayant 2 à 8 atomes de carbone, alcynyle ayant 2 à 8 atomes de carbone ou un groupe cycloakyle, cycloalkylalkyle, cycloalcényle ou cycloalcénylalkyle ayant chacun 3 à 8 atomes de carbone dans la partie cycloalkyle ou cycloalcényle et le cas échéant 1 à 6 atomes de carbone dans la partie alkyle linéaire ou ramifié, portant chacun le cas échéant dans la partie cycloalkyle ou cyclalcényle un ou plusieurs substituants identiques ou différents, en considérant dans chaque cas comme substituants de la partie cycloalkyle ou cycloalcényle :
un radical halogéno, hydroxy, un radical alkyle, alcanediyle, alkoxy, alkoxyalkyle, alkylthio ou dialkylamino linéaire ou ramifié dans chaque cas, ayant chacun 1 à 6 atomes de carbone dans les parties alkyle individuelles, ou un radical halogénalkyle, halogénalkoxy, halogénalkylthio linéaire ou ramifié dans chaque cas ayant chacun 1 à 4 atomes de carbone et 1 à 9 atomes d'halogènes identiques ou différents, en outre un radical arylalkyle, aryle, hétérocyclylalkyle ou hétérocyclyle portant chacun le cas échéant dans la partie aryle ou hétérocyclyle un ou plusieurs substituants identiques ou différents et ayant chaucn le cas échéant 6 à 10 atomes de carbone dans la partie aryle ou 2 à 9 atomes de carbone et 1 à 4 hétéroatomes identiques ou différents - en particulier azote, oxygène et/ou soufre - dans la partie hétérocyclyle et le cas échéant 1 à 6 atomes de carbone dans la partie alkyle linéaire ou ramifié, en considérant comme substituants de la partie aryle ou hétérocyclyle les substituants mentionnés dans chaque cas pour $R^1$, ou forment conjointement un noyau cycloalkyle ayant 3 à 7 atomes de carbone,

n représente le nombre 0, 1, 2 ou 3,

$X^1$ est de l'oxygène ou du soufre,

$X^2$ est de l'oxygène ou du soufre et

$X^3$ est de l'oxygène ou du soufre,

l'un au moins des substituants $X^1$, $X^2$ et/ou $X^3$ représentant toutefois du soufre.

**3.** Composés de formule (I) suivant la revendication 1, dans lesquels

$R^1$ représente un groupe, linéaire ou ramifié, alkyle ayant 1 à 6 atomes de carbone, alcényle ayant 2 à 6 atomes de carbone, alcynyle ayant 2 à 6 atomes de carbone, halogénalkyle ayant 1 à 4 atomes de carbone et 1 à 9 atomes d'halogènes identiques ou différents, halogénalcényle ayant 2 à 4 atomes de carbone et 1 à 7 atomes d'halogènes identiques ou différents, halogénalcynyle ayant 2 à 4 atomes de carbone et 1 à 7 d'halogènes identiques ou différents, cycloalkyle ayant 3 à 7 atomes de carbone, cycloalcényle ayant 3 à 7 atomes de carbone ou un groupe arylalkyle, aryle, hétéroarylalkyle ou hétéroaryle portant chacun le cas échéant dans la partie aryle ou hétéroaryle 1 à 5 substituants identiques ou différents et ayant chacun le cas échéant 6 à 10 atomes de carbone dans la partie aryle ou 2 à 9 atomes de carbone et 1 à 3 hétéroatomes identiques ou différents - notamment azote, oxygène et/ou soufre - dans la partie hétéroaryle et le cas échéant 1 à 4 atomes de carbone dans la partie alkyle linéaire ou ramifié, en considérant dans chaque cas comme substituants de la partie

aryle ou hétéroaryle :

un radical halogéno, cyano, nitro, hydroxy, formyle, hydroxycarbonyle, aminocarbonyle, aminosulfonyle, un radical alkyle, alkoxy, alkoxyalkyle, alkylthio, alkylsulfinyle, alkylsulfonyle, alkoxysulfonyle, alkylsulfonyloxy, ou dialkylamino linéaire ou ramifié dans chaque cas, ayant chacun 1 à 4 atomes de carbone dans les parties alkyle individuelles, un radical halogénalkyle, halogénalkoxy, halogénalkylthio, halogénalkylsulfinyle ou halogénalkylsulfonyle linéaire ou ramifié dans chaque cas, ayant chacun 1 à 4 atomes de carbone et 1 à 9 atomes d'halogènes identiques ou différents, un radical alkylcarbonyle, alkylcarbonyloxy, alkoxycarbonyle, N-alkylaminocarbonyle, N,N-dialkylaminocarbonyle, alkylcarbonylamino, alkylcarbonylaminoalkyle, ou alkoximinoalkyle linéaire ou ramifié dans chaque cas, ayant chacun 1 à 4 atomes de carbone dans les parties alkyle individuelles, ainsi qu'un radical phényle, phénoxy, phénylcarbonyle, phénoxycarbonyle ou phénylcarbonyloxy portant chacun le cas échéant dans la partie phényle un ou plusieurs substituants, identiques ou différents, halogéno et/ou alkyle linéaire ou ramifié ayant 1 à 4 atomes de carbone,

$R^2$ est de l'hydrogène, un groupe alkyle linéaire ou ramifié ayant 1 à 6 atomes de carbone ou un groupe cycloalkyle de 3 à 7 atomes de carbone,

$R^3$ est de l'hydrogène, un groupe alkyle linéaire ou ramifié ayant 1 à 6 atomes de carbone ou un groupe cycloalkyle ayant 3 à 7 atomes de carbone et

$R^4$ est un groupe alkyle linéaire ou ramifié ayant 1 à 6 atomes de carbone ou un groupe cycloalkyle ayant 3 à 6 atomes de carbone, ou bien

$R^3$ et $R^4$ forment conjointement avec l'atome de carbone auquel ils sont liés un noyau cyclalkyle de 3 à 7 chaînons,

$R^5$ représente un groupe cycloalkyle, cycloalkylalkyle, cycloalcénylalkyle ou cycloalcényle ayant chacun 3 à 7 atomes de carbone et portant chacun le cas échéant 1 à 5 substituants identiques ou différents, en considérant dans chaque cas comme substituants :

un radical halogéno, hydroxy, un radical alkyle, alcanediyle, alkoxy, alkoxyalkyle, alkylthio ou dialkylamino linéaire ou ramifié dans chaque cas, ayant chacun 1 à 4 atomes de carbone dans les parties alkyle individuelles, ou bien un radical halogénalkyle, halogénalkoxy, halogénalkylthio, linéaire ou ramifié dans chaque cas, ayant chacun 1 à 4 atomes de carbone et 1 à 9 atomes d'halogènes identiques ou différents, en outre un radical aryle ou arylalkyle de 6 à 10 atomes de carbone ou un radical hétérocyclyle ou hétérocyclylalkyle ayant 2 à 9 atomes de carbone et 1 à 3 hétéroatomes identiques ou différents - notamment azote, oxygène et/ou soufre - portant chacun le cas échéant 1 à 5 substituants identiques ou différents, en considérant dans chaque cas comme substituants ceux qui ont été mentionnés pour $R^1$,

$R^7$, $R^6$ représentent indépendamment l'un de l'autre de l'hydrogène, un groupe, linéaire ou ramifié dans chaque cas, alkyle ayant 1 à 6 atomes de carbone, cyanalkyle ayant 2 à 5 atomes de carbone, alcényle ayant 2 à 6 atomes de carbone, alcynyle ayant 2 à 6 atomes de carbone ou un groupe cycloalkyle, cycloalkylalkyle, cycloalcényle ou cycloalcénylalkyle portant chacun le cas échéant dans la partie cycloalkyle ou cycloalcényle 1 à 5 substituants identiques ou différents et ayant chacun 3 à 7 atomes de carbone dans la partie cycloalkyle ou cycloalcényle et le cas échéant 1 à 4 atomes de carbone dans la partie alkyle linéaire ou ramifié, en considérant comme substituants de la partie cycloalkyle ou cycloalcényle :

un radical halogéno, hydroxy, un radical alkyle, alcanediyle, alkoxy, alkoxyalkyle, alkylthio ou dialkylamino linéaire ou ramifié dans chaque cas, ayant chacun 1 à 4 atomes de carbone dans les parties alkyle individuelles ou un radical halogénalkyle, halogénalkoxy, halogénalkylthio linéaire ou ramifié dans chaque cas ayant chacun 1 à 4 atomes de carbone et 1 à 9 atomes d'halogènes identiques ou différents,

en outre un radical arylalkyle, aryle, hétérocyclylalkyle ou hétérocyclyle portant chacun le cas échéant dans la partie aryle ou hétérocyclyle de 1 à 5 substituants identiques ou différents et ayant chacun le cas échéant 6 à 10 atomes de carbone dans la partie aryle ou 2 à 9 atomes de carbone et 1 à 3 hétéroatomes identiques ou différents - notamment azote, oxygène et/ou soufre - dans la partie hétérocyclyle et le cas échéant 1 à 4 atomes de carbone dans la partie alkyle linéaire ou ramifié, en considérant dans chaque cas comme substituants de la partie aryle ou hétérocyclyle ceux qui ont été

mentionnés pour $R^1$,
ou forment ensemble un noyau cycloalkyle ayant 3 à 6 atomes de carbone,

n représente le nombre 0, 1, 2 ou 3,

$X^1$ est de l'oxygène ou du soufre,

$X^2$ est de l'oxygène ou du soufre et

$X^3$ est de l'oxygène ou du soufre,

l'un au moins des substituants $X^1$, $X^2$ et/ou $X^3$ représentant toutefois du soufre.

**4.** Composés de formule (I) suivant la revendication 1, dans lesquels

$R^1$ représente un groupe, linéaire ou ramifié dans chaque cas, alkyle ayant 2 à 5 atomes de carbone, alcényle ayant 2 à 4 atomes de carbone, alcynyle ayant 2 à 4 atomes de carbone, halogénalkyle ayant 1 ou 2 atomes de carbone et 1 à 5 atomes d'hydrogène identiques ou différents, halogénalcényle ayant 2 ou 3 atomes de carbone et 1 à 5 atomes d'halogènes identiques ou différents, halogénalcényle ayant 2 ou 3 atomes de carbone et 1 à 5 atomes d'halogènes identiques ou différents, cycloalkyle ayant 3 à 6 atomes de carbone, cycloalcényle ayant 3 à 6 atomes de carbone ou un groupe phénylalkyle ou phényle portant chacun le cas échéant dans la partie phényle 1 à 3 substituants identiques ou différents et ayant chacun le cas échéant 1 à 4 atomes de carbone dans la partie alkyle linéaire ou ramifié, en considérant dans chaque cas comme sustituants de la partie phényle : un radical halogéno, cyano, nitro, hydroxy, formyle, hydroxycarbonyle, aminocarbonyle, aminosulfonyle, un radical alkyle, alkoxy, alkoxyalkyle, alkylthio, alkylsulfinyle, alkylsulfonyle, alkoxysulfonyle, alkylsulfonyloxy ou dialkylamino linéaire ou ramifié dans chaque cas, ayant chacun 1 à 3 atomes de carbone dans les parties alkyle individuelles, un radical halogénalkyle, halogénalkoxy, halogénalkylthio, halogénalkylsulfinyle ou halogénoalkylsulfonyle linéaire ou ramifié dans chaque cas ayant chacun 1 ou 2 atomes de carbone et 1 à 5 atomes d'halogènes identiques ou différents, un radical alkylcarbonyle, alkylcarbonyloxy, alkoxycarbonyle, N-alkylaminocarbonyle, N,N-dialkylaminocarbonyle, alkylcarbonylamino, alkylcarbonylaminoalkyle ou alkoximinoalkyle linéaire ou ramifié dans chaque cas, ayant chacun 1 à 3 atomes de carbone dans les parties alkyle individuelles, ainsi qu'un radical phényle, phénoxy, phénylcarbonyle, phénoxycarbonyle ou phénylcarbonyloxy portant chacun le cas échéant dans la partie phényle un ou plusieurs substituants identiques ou différents halogéno et/ou alkyle linéaire ou ramifié ayant 1 à 3 atomes de carbone,

$R^2$ représente de l'hydrogène, un groupe alkyle linéaire ou ramifié ayant 1 à 4 atomes de carbone ou un groupe cycloalkyle ayant 3 à 6 atomes de carbone,

$R^3$ représente de l'hydrogène, un groupe alkyle linéaire ou ramifié ayant 1 à 4 atomes de carbone ou un groupe cycloalkyle ayant 3 à 6 atomes de carbone et

$R^4$ est un groupe alkyle linéaire ou ramifié ayant 1 à 4 atomes de carbone ou un groupe cycloalkyle de 3 à 6 atomes de carbone, ou bien

$R^3$ et $R^4$ forment, conjointement avec l'atome de carbone auquel ils sont liés, un noyau cycloalkyle de 3 à 6 chaînons,

$R^5$ est un groupe cycloalkyle, cycloalkylalkyle, cycloalcénylalkyle ou cycloalcényle portant chacun le cas échéant 1 à 3 substituants identiques ou différents et ayant chacun 3 à 6 atomes de carbone, en considérant dans chaque cas comme substituants du noyau cycloalkyle ou cycloalcényle :
un radical halogéno, hydroxy, un radical alkyle, alcanediyle, alkoxy, alkoxyalkyle, alkylthio ou dialkylamino linéaire ou ramifié dans chaque cas ayant chacun 1 à 3 atomes de carbone dans les parties alkyle individuelles, ou un radical halogénalkyle, halogénalkoxy, halogénalkylthio linéaire ou ramifié dans chaque cas ayant chacun 1 ou 2 atomes de carbone et 1 à 5 atomes d'halogènes identiques ou différents,
en outre un radical phényle ou phénylalkyle ou un radical hétérocyclyle ou hétérocyclylalkyle ayant 2 à 9 atomes de carbone et 1 à 3 hétéroatomes identiques ou différents - notamment azote, oxygène et/ou soufre, portant chacun le cas échéant 1 à 3 substituants identiques ou différents, en considérant dans chaque cas comme substituants du noyau phényle ou hétérocyclyle les substituants mentionnés pour $R^1$,

$R^7$, $R^6$ représentent, indépendamment l'un de l'autre, de l'hydrogène, un groupe linéaire ou ramifié dans chaque cas, alkyle ayant 1 à 4 atomes de carbone, cyanalkyle ayant 2 ou 3 atomes de carbone, alcényle ayant 2 à 4 atomes de carbone, alcynyle ayant 2 à 4

atomes de carbone ou un groupe cycloalkyle, cycloalkylalkyle, cycloalcényle ou cycloalcénylalkyle portant chacun le cas échéant dans la partie cycloalkyle ou cycloalcényle 1 à 3 substituants identiques ou différents et ayant chacun 3 à 6 atomes de carbone dans la partie cycloalkyle ou cycloalcényle et le cas échéant 1 à 3 atomes de carbone dans la partie alkyle linéaire ou ramifié, en considérant dans chaque cas comme substituants du noyau cycloalkyle ou cycloalcényle :

un radical halogéno, hydroxy, un radical alkyle, alcanediyle, alkoxy, alkoxyalkyle, alkylthio ou dialkylamino linéaire ou ramifié ayant chacun 1 à 3 atomes de carbone dans les parties alkyle individuelles ou un radical halogénalkyle, halogénalkoxy, halogénalkylthio, linéaire ou ramifié dans chaque cas ayant chacun 1 ou 2 atomes de carbone et 1 à 5 atomes d'halogènes identiques ou différents,

en outre un radical phénylalkyle, phényle, hétérocyclylalkyle ou hétérocyclyle portant chacun le cas échéant dans la partie phényle ou hétérocyclyle 1 à 3 substituants identiques ou différents et ayant chacun le cas échéant 2 à 9 atomes de carbone et 1 à 3 hétéroatomes identiques ou différents - notamment azote, oxygène et/ou soufre, dans la partie hétérocyclyle et le cas échéant 1 à 3 atomes de carbone dans la partie alkyle linéaire ou ramifié, en considérant dans chaque cas comme substituants de la partie aryle ou hétérocyclyle les substituants qui ont été mentionnés pour $R^1$, ou forment conjointement un noyau cycloalkyle de 3 à 5 atomes de carbone,

n représente le nombre 1 ou 2,

$X^1$ est de l'oxygène ou du soufre,

$X^2$ est de l'oxygène ou du soufre et

$X^3$ est de l'oxygène ou du soufre,

l'un au moins des substituants $X^1$, $X^2$ et/ou $X^3$ représentant toutefois du soufre.

**5.** Compositions pesticides, caractérisées par une teneur en au moins un amide d'aminoacides de formule (I) suivant la revendication 1.

**6.** Procédé pour combattre des parasites, caractérisé en ce qu'on fait agir des amides d'aminoacides de formule (I) suivant la revendication 1 sur des parasites et/ou sur leur milieu.

**7.** Procédé de production d'amides d'aminoacides de formule générale (I)

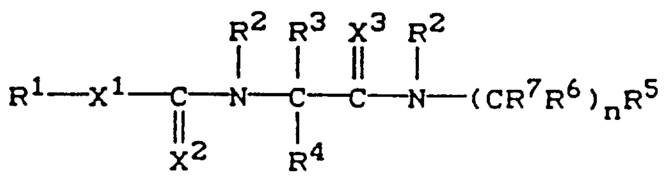

(I)

dans laquelle

n, $R^1$, $R^2$, $R^3$, $R^4$, $R^5$, $R^6$, $R^7$, $X^1$, $X^2$ et $X^3$ ont la définition indiquée dans la revendication 1, caractérisé en ce qu'on fait réagir éventuellement en présence d'un diluant et en la présence éventuelle d'une substance auxiliaire de réaction, des aminoacides substitués de formule (II)

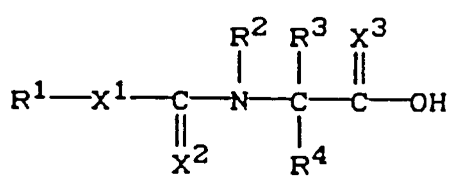

(II)

dans laquelle

$R^1$, $R^2$, $R^3$, $R^4$, $X^1$, $X^2$ et $X^3$ ont la définition indiquée ci-dessus,

avec des amines de formule (III),

$R^2HN-(CR^7R^6)_nR^5$ (III)

dans laquelle
n, $R^2$, $R^5$, $R^6$ et $R^7$ ont la définition indiquée ci-dessus.

**8.** Utilisation d'amides d'aminoacides de formule (I) suivant les revendications 1 à 7 pour combattre des parasites.

**9.** Procédé de préparation de compositions pesticides, caractérisé en ce qu'on mélange des amides d'aminoacides de formule (I) suivant les revendications 1 à 7 avec des diluants et/ou des agents tensioactifs.